# DEMANDE DE BREVET EUROPEEN

(11) **EP 0 532 410 A1**
(43) Date de publication de la demande: **17.03.1993**
(21) Numéro de dépôt: 92402459.9
(22) Date de dépôt: 09.09.1992
(51) Int. Cl.: C07D 233/70, A61K 31/415, C07D 233/84, C07D 239/22, C07D 403/10, C07D 417/12, A61K 31/505, A61K 31/425

(54) **Dérivés hétérocycliques N-substitués, comme inhibiteurs de l'angiotensive II**

(30) Priorité: 10.09.1991 FR 9111161
(71) Demandeur: SANOFI, F-75008 Paris (FR)
(72) Inventeur: Perreaut, Pierre, F-34980 Saint Gely Du Fesc (FR); Muneaux, Claude, F-34270 Les Matelles (FR); Muneaux, Yvette, F-34270 Les Matelles (FR)
(74) Mandataire: Gillard, Marie-Louise

(57) **Abrégé**

L'invention a pour objet des dérivés hétérocycliques N-substitués de formule**:** 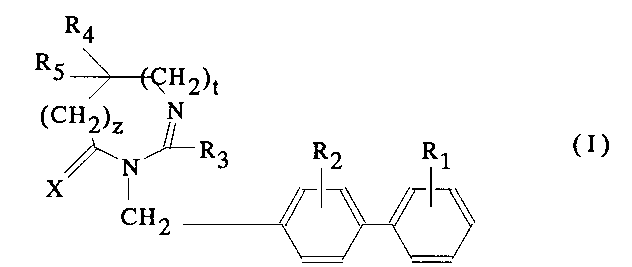
dans laquelle**:**
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyano-acétamide, un N-hydroxy-acétamide, un N-((carboxy-4) thiazol- 1,3-yl-2 acétamide, un ureido, un cyano-2 guanidinocarbonyle, un cyano-2 guanidinométhyle, un imidazol-1-yl-carbonyle, un cyano-3 méthyl-2 isothioureidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄;
- R₄ représente un alkyle en C₁-C₆, non substitué ou substitué par un ou plusieurs atomes d'halogène ;
- R₅ représente un cycloalkyle ou un cycloalkylméthyle, ledit cycloalkyle étant en C₃-C₇ non substitué ou substitué par un ou plusieurs atomes d'halogène ;
- ou R₄ et R₅ représentent chacun un cyclopropyle ;
- X représente un atome d'oxygène ou un atome de soufre ;
- z et t sont nuls ou l'un est nul et l'autre représente un ;

et ses sels.

Application : Antagonistes de l'angiotensine II

## Description

La présente invention concerne des dérivés hétérocycliques N-substitués, leur préparation et les compositions pharmaceutiques en contenant.

Les composés selon l'invention antagonisent l'action de l'angiotensine II qui est une hormone peptidique de formule :
H-Asp-Arg-Val-Tyr-Ile-His-Pro-Phe-OH

L'angiotensine II est un puissant agent vasopresseur qui est le produit biologiquement actif du système rénine-angiotensine : la rénine agit sur l'angiotensinogène du plasma pour produire l'angiotensine I, celle-ci est convertie en angiotensine II par action de l'enzyme de conversion de l'angiotensine I.

Les composés de la présente invention sont des composés non peptidiques, antagonistes de l'angiotensine II. En inhibant l'action de l'angiotensine II sur ses récepteurs, les composés selon l'invention empêchent notamment l'augmentation de la pression sanguine produite par l'interaction hormone-récepteur, ils ont également d'autres actions physiologiques au niveau du système nerveux central et au niveau rénal, par exemple.

Ainsi les composés selon l'invention sont utiles dans le traitement d'affections cardiovasculaires comme l'hypertension, la défaillance cardiaque ainsi que dans le traitement d'affections du système nerveux central et dans le traitement du glaucome, de la rétinopathie diabétique et de l'insuffisance rénale.

De nombreux brevets ou demandes de brevets décrivent des composés non peptidiques ayant une activité antagoniste de l'Angiotensine II On peut signaler en particulier la demande de brevet EP 475898 publiée le 18.03.92 qui concerne de manière générale des composés de formule : 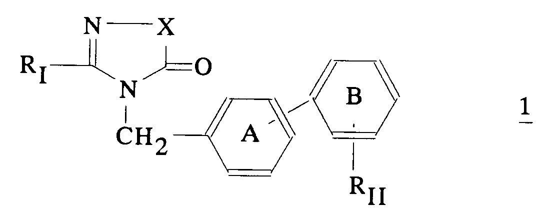
dans laquelle notamment,
- X représente un groupe de formule :

   -C(R_{III}R_{IV}-[C(R_{V})R_{VI}]ₚ-[C(R_{VII})R_{VIII}]_{q}-
- R_{I} représente un alkyle ou un alcényle non substitué ou substitué par un halogène,
- R_{II} représente un carboxy ou un tétrazolyle,
- R_{III} et R_{IV} représentent chacun indépendamment un alkyle non substitué ou substitué par un ou plusieurs halogènes, un cycloalkyle ou un reste aromatique.

La demande EP 475898 ne décrit pas spécifiquement des composés 1 dans lesquels R_{III} serait un alkyle et R_{IV} serait un cycloalkyle.

La présente invention a pour objet des composés de formule : 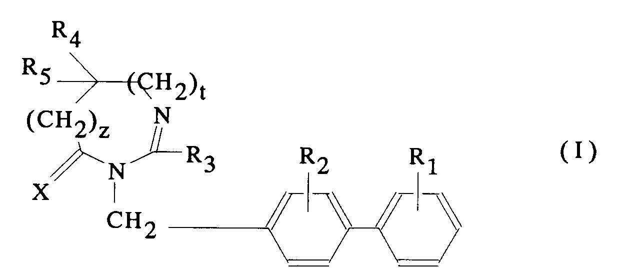
danslaquelle :
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyano-acétamide, un N-hydroxy-acétamide, un N-((carboxy-4) thiazol-1,3-yl-2 acétamide, un ureido, un cyano-2 guanidinocarbonyle, un cyano-2 guanidinométhyle, un imidazol-1-yl-carbonyle, un cyano-3 méthyl-2 isothioureidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ représente un alkyle en C₁-C₆, non substitué ou substitué par un ou plusieurs atomes d'halogène ;
- R₅ représente un cycloalkyle ou un cycloalkylméthyle, ledit cycloalkyle étant en C₃-C₇ non substitué ou substitué par un ou plusieurs atomes d'halogène ;
- ou R₄ et R₅ représentent chacun un cyclopropyle ;
- X représente un atome d'oxygène ou un atome de soufre ;
- z et t sont nuls ou l'un est nul et l'autre représente un ;

et leurs sels.

Lorsqu'un composé selon l'invention présente un carbone asymétrique, l'invention comprend chacun des 2 isomères optiques et leur mélange racémique.

Les sels des composés de formule (I) selon la présente invention comprennent ceux avec des acides minéraux ou organiques qui permettent une séparation ou une cristallisation convenable des composés de formule (I), tels que l'acide trifluoroacétique, l'acide picrique, l'acide oxalique ou un acide optiquement actif, par exemple un acide mandélique ou un acide camphosulfonique, et ceux qui forment des sels pharmaceutiquement acceptables tels que le chlorhydrate, le bromhydrate, le sulfate, l'hydrogénosulfate, le dihydrogénophosphate, le méthanesulfonate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine tertiaire, telle que le trométamol, ou bien les sels d'arginine, de lysine, ou de toute amine physiologiquement acceptable.

Selon la présente description et dans les revendications qui vont suivre, par alkyle on entend un radical d'hydrocarbure droit ou ramifié ; par atome d'halogène on entend un atome de brome, de chlore ou de fluor ; par groupe N-protecteur (également désigné par Pr) on entend un groupe utilisé classiquement dans la chimie des peptides pour permettre une protection temporaire de la fonction amine, par exemple un groupe Boc, Z, Fmoc ou un groupe benzyle ; par groupe carboxy estérifié on entend un ester labile dans les conditions appropriées, comme par exemple un ester méthylique, éthylique, benzylique ou tertiobutylique.

Les composés de formule (I) dans laquelle R₁ est en position ortho et représente un groupe carboxy ou tétrazolyle et R₂ est l'hydrogène sont des composés préférés.

Les composés de formule (I) dans lesquels R₄ est le méthyle et R₅ est le cyclohexyle, sont des composés préférés.

De même les composés de formule (I) dans lesquels R₃ représente un groupe alkyle droit en C₁-C₆ sont des composés préférés.

Les composés de formule (I) dans lesquels X représente un atome d'oxygène sont également des composés préférés.

Enfin les composés de formule (I) dans lesquels z = t = 0 sont des composés préférés.

Le n-butyl-2 méthyl-4 cyclohexyl-4 [((tétrazol-5-yl)-2′biphényl-4-yl) méthyl]-1 imidazoline-2 one-5 ou l'un de ses sels avec des acides ou des bases est particulièrement préféré.

Les abréviations suivantes sont utilisées dans la description et dans les exemples :
- Alcool: : Alcool éthylique
- Et: : Ethyl
- Carboglace: : dioxyde de carbone solide
- nBu, tBu: : n-butyl, *tert*-butyl
- DMF: : diméthylformamide
- THF: : tétrahydrofuranne
- DCM: : dichlorométhane
- NBS: : N-bromosuccinimide
- DCC: : dicyclohexylcarbodiimide
- DIPEA: : diisopropyléthylamine
- Ether: : éther éthylique
- TFA: : acide trifluoroacétique
- Z: : benzyloxycarbonyle
- Boc: : *tert*-butoxycarbonyle
- BOP: : hexafluorophosphate de benzotriazolyloxy trisdiméthylamino phosphonium
- Fmoc: : fluorenylméthyloxycarbonyle

Réactif de Lawesson : bis(methoxy-4 phényl)-2,4 dithia-1,3 diphosphetane-2,4 disulfure-2,4].

La présente invention a également pour objet le procédé de préparation des composés (I). Ledit procédé est caractérisé en ce que :
a1) on fait réagir un dérivé hétérocyclique de formule : 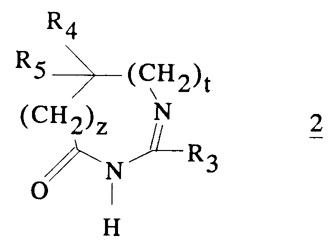 dans laquelle, z, t, R₃, R₄ et R₅ ont les significations indiquées ci-dessus pour (I), sur un dérivé du (biphényl-4-yl) méthyle de formule : 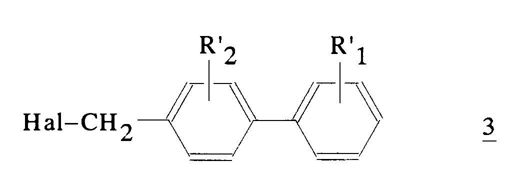 dans laquelle Hal représente un atome d'halogène et R′₁ et R′₂ représentent respectivement soit R₁ et R₂, soit un précurseur de R₁ et/ou R₂;
b1) éventuellement, le composé, ainsi obtenu de formule : 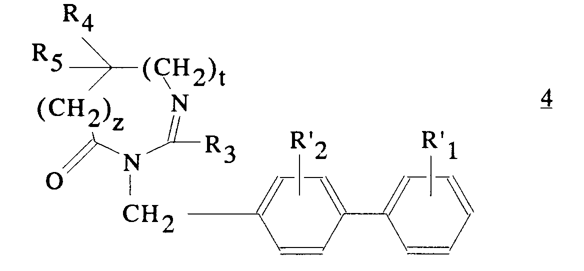 est traité par le réactif de Lawesson [bis(méthoxy-4 phényl)-2,4 dithia-1,3 diphosphétane-2,4 disulfure-2,4] ;
c1) le composé obtenu en a1) ou en b1), de formule : 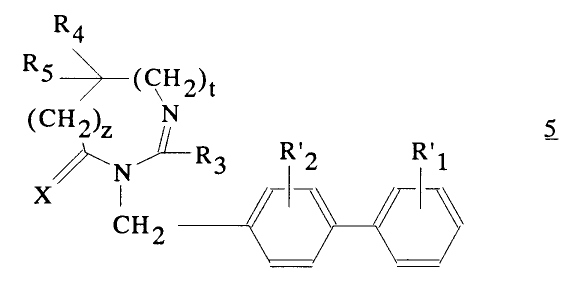 dans laquelle X représente un atome d'oxygène ou un atome de soufre, est traité pour préparer le composé (I) par transformation des groupes R′₁ et/ou R′₂ en respectivement R₁ et/ou R₂.
d2) éventuellement, le composé ainsi obtenu est transformé en l'un de ses sels selon des procédés classiques bien connus de l'homme de métier.

Parmi les composés 2 certains sont connus dans l'art antérieur, d'autres sont nouveaux et font partie de l'invention.

La demande de brevet EP 144748 décrit des herbicides de formule : 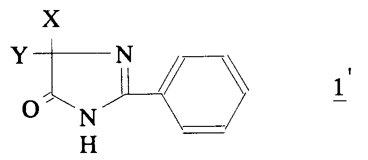
dans laquelle X et Y peuvent représenter l'un un alkyle et l'autre un cycloalkyle et le phényle est éventuellement substitué.

Ainsi la présente invention a également pour objet les composés (II) de formule : 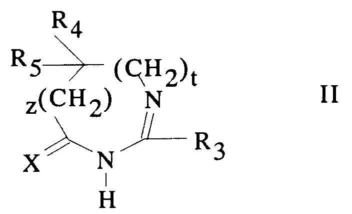
dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄;
- R₄ représente un alkyle en C₁-C₆, non substitué ou substitué par un ou plusieurs atomes d'halogène ;
- R₅ représente un cycloalkyle ou un cycloalkylméthyle, le cycloalkyle étant en C₃-C₇, non substitué ou substitué par un ou plusieurs atomes d'halogène ;
- ou encore R₄ et R₅ représentent chacun un cyclopropyle ;
- X représente un atome d'oxygène ;
- z et t sont nuls ou l'un est nul et l'autre représente un ;

avec la limitation que R₃ est autre que phényle ou phényle substitué lorsque simultanément z=t=0, R₅ représente un cycloalkyle.

Parmi les dérivés (II), les composés dans lesquels z=t=0, de formule : 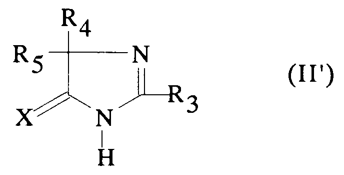
dans laquelle X, R₃, R₄ et R₅ ont les définitions données ci-dessus pour (II), avec la limitation que R₃ est autre que phényle, ou phényle substitué lorsque R₅ est un cycloalkyle sont des composés préférés. Particulièrement préférés sont les composés (II′) dans lesquels R₄ est un méthyle, R₅ est un cyclohexyle, R₃ est un alkyle en C₁-C₆.

Les composés (II) dans lesquels z=0 et t=1 de formule : 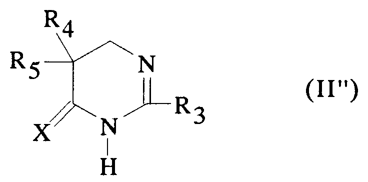
dans laquelle R₃, R₄, R₅ et X ont les définitions données ci-dessus pour (II), sont des composés préférés.

Enfin, les composés (II) dans lesquels z=1 et t=0 de formule : 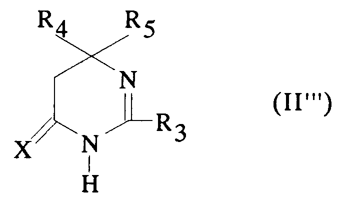
dans laquelle :
- R₃, R₄, R₅ et X ont les définitions données ci-dessus pour (II), sont des composés préférés.

Les dérivés 2 sont préparés par des méthodes connues. Par exemple, on peut utiliser la méthode décrite par jacquier et al. (Bull. Soc. Chim. France, 1971, (3), 1040-1051) et par Brunken et Bach (Chem. Ber., 1956, 89, 1363-1373) et faire agir un imidate d'alkyle sur un acide aminé ou son ester, selon le schéma réactionnel suivant : 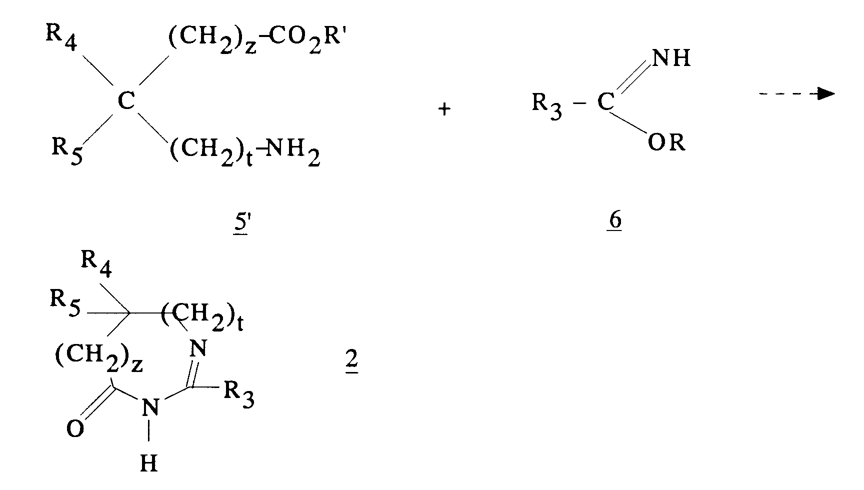
dans lequel R représente un alkyle en C₁-C₄ et R′ représente l'hydrogène ou un alkyle en C₁-C₄, et R₃, R₄, R_{5,} z et t, sont tels que définis précédemment pour (I).

Cette réaction est effectuée en milieu acide, par chauffage dans un solvant inerte tel que le xylène ou le toluène.

Les composés 5′ sont des composés connus ou préparés par des méthodes connues. Les composés 5′ peuvent être obtenus optiquement purs en utilisant des méthodes de synthèse asymétrique ou de résolution du mélange racémique telles que décrites dans Synthesis of Optically Active alpha-aminoacids, R.M. Williams, Pergamon Press, 1989.

Selon un autre mode opératoire, un composé 2 peut être préparé par action en milieu acide d'un aminoalkylamide (5˝) sur un ortho-ester d'alkyle (10) selon le schéma réactionnel suivant :

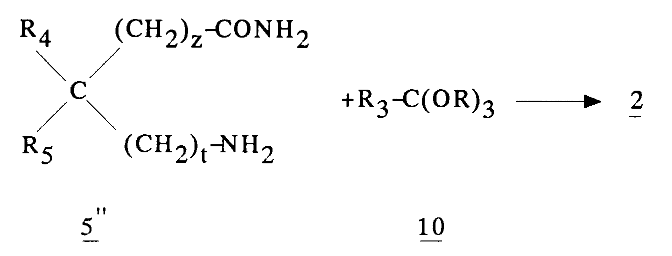

R représente un alkyle en C₁-C₄.

En utilisant un mode opératoire décrit par H. Takenaka et al. (Heterocycles, 1989, 29 (6), 1185-89), on peut également préparer le composé 2, en faisant agir sur le dérivé 5˝ un halogénure d'acide de formule : 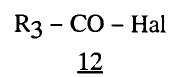
dans laquelle Hal représente un halogène, de préférence le chlore ; puis la cyclisation du diamide formé est réalisée en milieu basique.

Lorsque z=t=0, la préparation d'un dérivé 5˝ peut être effectuée à partir d'une cétone selon un mode opératoire décrit dans le brevet américain 4 017510 ou dans le brevet suisse 540271 :

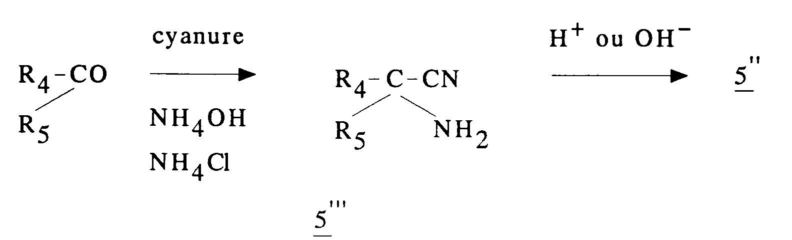

La cétone R₄R₅CO est traitée par du cyanure (acide cyanhydrique, cyanure de sodium ou cyanure de potassium) et des ions ammonium (ammoniaque et chlorure d'ammonium) ; le composé aminonitrile 5‴ est ensuite hydrolysé en 5˝, soit en milieu acide, soit en milieu basique.

Le cas échéant, on peut résoudre l'aminonitrile 5‴ par un acide optiquement actif, selon le mode opératoire décrit dans le brevet européen 158000. On peut alors préparer le composé 5˝ puis le composé 2 sous forme optiquement pure.

Plus particulièrement, un composé 2, selon un autre objet de la présente invention, est préparé par un procédé caractérisé en ce que l'on fait agir sur un composé de formule : 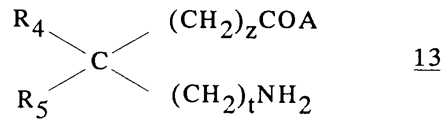
dans laquelle A représente un groupe OH, un groupe NH₂ ou un groupe OR′, R′ étant l'hydrogène ou un alkyle en C₁-C₄, un composé de formule :

R₃ - B 14

dans laquelle B représente :
- un groupement C (OR)₃
- un groupement 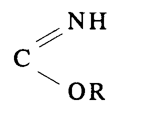 ou
- un groupement COHal ;

R étant un alkyle en C₁-C₄ et Hal désignant un atome d'halogène, de préférence le chlore.

Le dérivé du (biphényl-4-yl) méthyle (3) est préparé selon un procédé décrit dans la demande de brevet EP 324 377.

La transformation d'un groupe R′₁ et/ou R′₂ en un groupe R₁ et/ou R₂ est effectuée par des méthodes bien connues de l'homme de l'art. Ainsi, lorsque le composé (I) à préparer possède un groupe R₁ et/ou R₂=carboxy, R′₁ et/ou R′₂ représente un groupe carboxy estérifié. Lorsque le composé (I) à préparer possède un groupe R₁ et/ou R₂=tétrazolyle, R′₁ et/ou R′₂ peut représenter, soit un tétrazolyle protégé par exemple par un groupe trityle, soit un groupe cyano qui sera ensuite remplacé par un groupe tétrazolyle éventuellement protégé par un trityle. La transformation du groupe cyano en un tétrazolyle peut être effectuée par un azide, par exemple, l'azide de tributyl étain ou par l'azide de sodium.

On peut également utiliser des groupes R′₁ et/ou R′₂ tels que les groupes nitro, carboxy, cyano ou chlorure d'acide et les transformer ensuite par des réactions bien connues de l'homme de l'art pour obtenir des groupes R₁ et/ou R₂ tels que définis pour le composé I.

Ainsi, lorsque R′₁ et/ou R′₂ représente un carboxy, il peut être transformé en R₁ et/ou R₂ représentant un imidazol-1-yl carbonyle, ou bien en N-[(carboxy-4 thiazol-1,3 yl-2)]acétamide

Le groupe R′₁ et/ou R′₂ représentant un chlorure d'acide peut être transformé en R₁ et/ou R₂ représentant N-hydroxy-acétamide, N-cyano-acétamide, uréido ou cyano-2 guanidinocarbonyle.

Le groupe R′₁ et/ou R′₂ représentant un nitro peut être transformé en amino à partir duquel on prépare R₁ et/ou R₂, tel que méthylsulfonylamino, trifluorométhylsulfonylamino et trifluorométhylsulfonylaminométhyle.

Le groupe R′₁ et/ou R′₂ représentant un cyano peut être transformé en aminométhyle à partir duquel on prépare un cyano-3 méthyl-2 isothiouréidométhyle (selon C. Gordon et al., J. Org. Chem., 1970, 35 (6), 2067-2069), un cyano-2 guanidinométhyl (selon R.W. Turner, Synthesis, 1975, 332).

L'étape a1) est réalisée dans un solvant tel que le DMF, le DMSO ou le THF, en milieu basique, par exemple en présence de potasse, d'un alcoolate métallique ou d'un hydrure métallique.

L'étape b1) est réalisée par chauffage sous azote dans un solvant tel que le toluène, selon la méthode décrite par M.P. Cava et al., Tetrahedron, 1985, 41, 22, 5061.

Dans la description ci-après, le procédé comprenant les étapes a1), b1) et c1), est appelé procédé l.

Alternativement, les composés (I) peuvent être préparés selon un autre procédé qui est également un objet de la présente invention. Ce procédé est caractérisé en ce que :
a2) on fait réagir un aminoacide de formule : 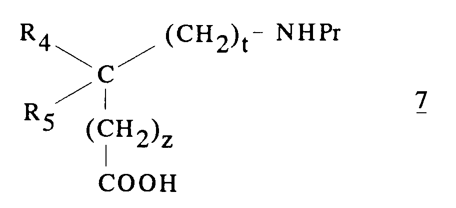 dans laquelle z, t, R₄ et R₅ ont les significations indiquées ci-dessus pour (I) et dont la fonction amine est protégée par le groupe Pr, sur un dérivé (biphényl-4-yl) méthylamine de formule : 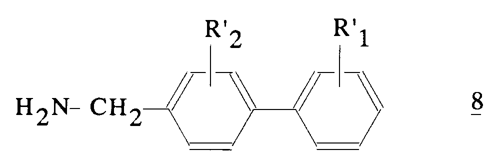 dans laquelle R′₁ et R′₂ représentent respectivement soit R₁ et R₂, soit un groupement précurseur de R₁ et R₂ ;
b2) après déprotection de l'amine, le composé ainsi obtenu de formule : 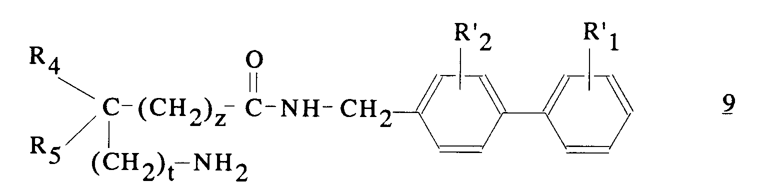 est ensuite traité par un ortho-ester d'alkyle de formule R₃C(OR)₃ (10), dans laquelle R₃ a la signification indiquée ci-dessus pour (I) et R est un alkyle en C₁-C₄ ;
c2) éventuellement, le composé ainsi obtenu de formule : 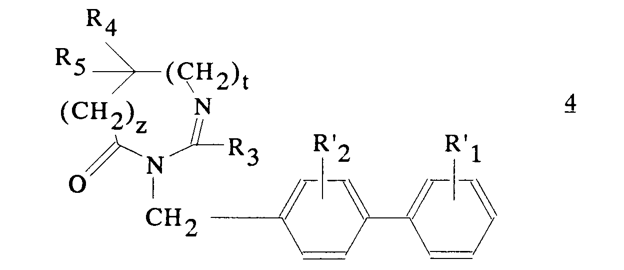 est traité par le réactif de Lawesson [bis(méthoxy-4 phényl)-2,4 dithia-1,3 diphosphénate-2,4 disulfure 2,4] ;
d2) le composé ainsi obtenu en b2) ou en c2) de formule : 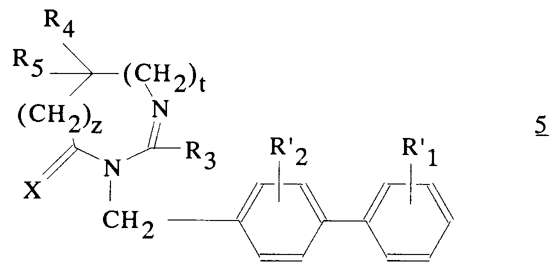 est ensuite traité dans des conditions convenables pour préparer le composé (I) par transformation des groupes R′₁ et/ou R′₂ en, respectivement, les groupes R₁ et/ou R₂ ;
e2) le composé ainsi obtenu est éventuellement transformé en l'un de ses sels.

Les composés 7 sont connus ou préparés par des méthodes connues (Chemistry of the Amino Acids, Greenstein and Winitz, John Wilez ed., 1961, vol.I, p.697). Eventuellement, ces composés peuvent être obtenus optiquement purs en utilisant des méthodes de synthèse asymétrique ou de résolution du mélange racémique, telles que décrites dans "Synthesis of Optically Active alpha-aminoacids" R.M. Williams, Pergamon Press, 1989.

Les composés 8 sont préparés selon la demande de brevet européen 324 377. L'étape a2) est réalisée dans les conditions habituelles du couplage d'un acide sur une amine, par exemple en présence de BOP et de DIPEA.

L'étape b2) qui est la cyclisation du composé 9 en présence de 10 est effectuée selon Jacquier et al. (Bull. Soc. Chim. France, 1971, (3), 1040-1051) et selon Brunken et Bach (Chem. Ber., 1956, 89, 1363-1373).

Dans la description ci-après, le procédé comprenant les étapes a2) à d2) est appelé procédé 2.

Selon une variante du procédé 2, à l'étape b2), on peut éventuellement isoler un intermédiaire 9′ de formule : 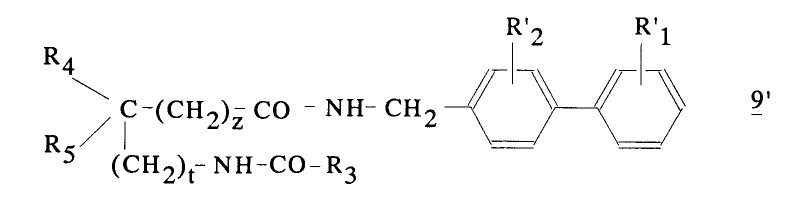
puis préparer le composé 4 par cyclisation en milieu acide ou basique.

L'affinité des produits selon l'invention pour les récepteurs de l'angiotensine II a été étudiée sur un test de liaison de l'angiotensine II marquée à l'iode 125 à des récepteurs membranaires de foie de rats. La méthode utilisée est celle décrite par S. Keppens et al. dans Biochem. J., 1982, 208, 809-817.

On mesure la CI₅₀ : concentration qui donne 50% de déplacement de l'angiotensine II marquée, liée spécifiquement au récepteur. La CI₅₀ des composés selon l'invention est inférieure à 10⁻⁶M.

De plus, l'effet antagoniste de l'angiotensine II des produits selon l'invention a été constaté sur différentes espèces animales dans lesquelles le système rénine-angiotensine a été préalablement activé (C. Lacour et al., J. Hypertension, 1989, 7 (suppl.2), S33-S35).

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement de différentes affections cardiovasculaires, notamment l'hypertension, la défaillance cardiaque, l'insuffisance veineuse, l'insuffisance rénale, ainsi que dans le traitement du glaucome, des rétinopathies diabétiques et de différentes affections du système nerveux central, l'anxiété, la dépression, les déficits mnésiques ou la maladie d'Alzheimer par exemple.

La présente invention a également pour objet des compositions pharmaceutiques contenant une dose efficace d'un composé selon l'invention ou d'un sel pharmaceutiquement acceptable et des excipients convenables. Lesdits excipients sont choisis selon la forme pharmaceutique et le mode d'administration souhaités.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule I ci-dessus, ou leurs sels éventuels, peuvent être administrés sous formes unitaires d'administration, en mélange avec des supports pharmaceutiques classiques aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire, ou intraveineuse, et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,1 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose d'un dérivé cellulosique ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir, ou pour l'administration sous forme de gouttes, peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions saline isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un de leurs sels pharmaceutiquement acceptables, d'autres principes actifs tels que, par exemple, des tranquillisants ou d'autres médicaments qui peuvent être utiles dans le traitement des troubles ou maladies indiquées ci-dessus.

Ainsi, la présente invention a pour objet des compositions pharmaceutiques contenant plusieurs principes actifs en association dont l'un est un composé selon l'invention et le ou les autres peuvent être un composé béta-bloquant, un antagoniste calcique, un diurétique, un antiinflammatoire non stéroïdien ou un tranquillisant.

Les exemples suivants illustrent l'invention. Dans ces exemples, on utlise les abréviations suivantes :
d signifie densité, TA signifie température ambiante, KHSO₄-K₂SO₄ signifie une solution aqueuse contenant 16,6 g de bisulfate de potassium et 33,3 g de sulfate de potassium pour 1 litre.

Les points de fusion (Fc) sont donnés en degrés Celsius ; sauf indication contraire, ils sont mesurés sans recristallisation du produit.

La pureté des produits est vérifiée en chromatographie sur couche mince (CCM) ou par HPLC. Les produits sont caractérisés par leur spectre RMN enregistré à 200 MHz dans le DMSO deutéré, la référence interne étant le tétraméthylsilane.

Les pouvoirs rotatoires spécifiques (α_{D}) sont mesurés à 22°C : longueur de la cuve : 10 cm, concentration : 1 g pour 100 ml.

Pour l'interprétation des spectres de RMN, on emploie :
- s: pour singulet
- s.e.: pour singulet élargi
- d: pour doublet
- t: pour triplet
- q: pour quadruplet
- quint: pour quintuplet
- sext: pour sextuplet
- m: pour massif ou multiplet

Par ailleurs, im signifie imidazole.

### EXEMPLE 1

Trifluoroacétate de (R,S) n-butyl-2 [(carboxy-2′ biphényl-4-yl) méthyl]-1 cyclohexyl-4 méthyl-4 imidazoline-2 one-5.
A) Cyclohexyl-5 méthyl-5 hydantoïne.
   Ce composé est préparé selon J. Org. Chem., 1960, 25, 1920-1924.
   50 g de cyclohexylméthylcétone, dissous dans 400 ml d'alcool 95, sont ajoutés en 30 minutes à 29,4 g de cyanure de sodium et 192 g de carbonate d'ammonium dans 400 ml d'eau. On chauffe à 55-60°C pendant 4 heures puis on évapore de moitié sous vide et on laisse une nuit à +4°C. Le précipité formé est filtré, lavé à l'eau, puis séché sous vide sur anhydride phosphorique. On obtient 65,5 g de l'hydantoïne attendue, identifiée par ses spectres IR et RMN.
   Fc = 220°C
B) Acide (R,S) amino-2 cyclohexyl-2 propionique.
   Ce composé est préparé selon J. Org. Chem. 1960, 25, 1920-1924.
   Un mélange contenant 7 g de l'hydantoïne, préparée à l'étape précédente et 28 g d'octahydrate d'hydroxyde de baryum dans 150 ml d'eau, est porté à 160°C pendant 5 heures, dans un tube d'acier. Le milieu réactionnel est saturé avec de la carboglace, l'insoluble formé est filtré, puis le filtrat est concentré sous vide. Le résidu est repris dans de l'acétone, filtré et séché. On obtient 5,25 g de l'acide attendu, identifié par ses spectres IR et RMN. Le produit fond en se décomposant à 350°C.
C) Ester éthylique de l'acide (R,S) amino-2 cyclohexyl-2 propionique.
   3 g de l'acide préparé à l'étape précédente sont ajoutés à 40 ml d'alcool absolu saturé en gaz chlorhydrique, puis on chauffe à reflux, sous agitation, pendant 20 heures. Le milieu réactionnel est évaporé sous vide et le résidu est repris dans un mélange éther-eau que l'on amène à pH9 par addition d'une solution saturée de bicarbonate de sodium. La phase organique est décantée, lavée par une solution saturée de chlorure de sodium, puis évaporée sous vide. On obtient 2,1 g de l'ester attendu sous forme d'huile. Identification par spectres IR et RMN.
D) Valérimidate d'éthyle.
   Ce composé est préparé sous forme de chlorhydrate selon Mac Elvain (J. Amer. Chem. Soc., 1942, 64, 1825-1827). Il est libéré de son chlorhydrate par action du carbonate de potassium, puis extrait par le DCM.
E) (R,S) n-butyl-2 cyclohexyl-4 méthyl-4 imidazoline-2 one-5.
   2 g de l'ester préparé à l'étape C) et 2,35 g de valérimidate d'éthyle sont mélangés dans 6 ml de xylène auxquels on ajoute 6 gouttes d'acide acétique ; on porte à reflux pendant 6 heures. Le milieu réactionnel est ensuite concentré sous vide et le résidu est chromatographié sur gel de silice fine en éluant par un mélange chloroforme/méthanol/ acide acétique (95/9/3 ; v/v/v). Les fractions contenant le produit désiré sont rassemblées puis évaporées sous vide ; le résidu est repris par un mélange acétate d'éthyle/eau et on amène à pH9 par addtion d'une solution de soude. La phase organique est décantée, lavée à l'eau, puis par une solution saturée de chlorure de sodium, séchée sur sulfate de sodium, puis évaporée à sec. On obtient le produit attendu sous forme d'une huile épaisse qui se prend en masse pour donner un solide amorphe.
   m = 1,56 g
   IR (chloroforme) :
   1720 cm⁻¹ C=O
   1640 cm⁻¹ C=N
   RMN conforme
F) Bromométhyl-4 *ter*-butoxycarbonyl-2′ biphényle.
   Ce composé est préparé selon le procédé décrit dans la demande de brevet européen 324 377.
G) (R,S) [(n-butyl-2 cyclohexyl-4 méthyl-4 [(*tert*-butoxycarbonyl-2' biphényl-4-yl) méthyl]-1 imidazoline-2 one-5.
   1,5 g de l'imidazolinone préparée à l'étape précédente est dissous dans 20 ml de DMF. On ajoute 250 mg d'hydrure de sodium à 80% dans l'huile. Après 20 minutes d'agitation, on ajoute 2,48 g du composé préparé à l'étape F) et on laisse 2 heures à TA. Le milieu réactionnel est repris par un mélange acétate d'éthyle/eau ; la phase aqueuse est décantée, lavée par de l'eau, par une solution saturée de chlorure de sodium, séchée sur sulfate de sodium, puis concentrée sous vide. Le résidu est chromatographié sur silice en éluant par un mélange acétate d'éthyle/toluène (1/4 ; v/v). On obtient 1,8 g du produit attendu sous forme de cire blanche.
   IR (chloroforme)
   1710-1730 cm⁻¹ C=O ester et imidazoline
   1630 cm⁻¹ C=N
H) Trifluoroacétate de (R,S) n-butyl-2 [(carboxy-2′ biphényl-4-yl) méthyl]-1 cyclohexyl-4 méthyl-4 imidazoline-2 one-5.

1,5 g du composé obtenu à l'étape précédente est agité pendant 40 minutes dans 7 ml de TFA et 7 ml de DCM. Le milieu réactionnel est concentré sous vide et repris dans de l'éther. On obtient un solide blanc qui est filtré, lavé à l'éther et séché sous vide.
m = 1,40 g
Fc = 171°C
MH⁺ : 447
RMN :
7,10-7,70 ppm : m : 8 H aromatiques
4,45 ppm : s : 2 H : N-CH₂-C₆H₄-
1,25 ppm : s : CH₃ en 4

### EXEMPLE 2

Trifluoroacétate de (R,S) n-butyl-2 [(carboxy-2′ biphényl-4-yl) méthyl]-1 cyclohexylméthyl-4 méthyl-4 imidazoline-2 one-5.

En procédant selon les modes opératoires décrits à l'exemple 1, on prépare à partir de la cyclohexylméthylcétone les composés suivants :
A) Cyclohexylméthyl-5 méthyl-5 hydantoïne.
   Fc = 205-206°C
B) Acide (R,S) amino-2 cyclohexyl-3 méthyl-2 propionique.
   Caractérisé par ses spectres IR et RMN.
C) ester éthylique de l'acide (R,S) amino-2 cyclohexyl-3 méthyl-2 propionique.
   Caractérisé par ses spectres IR et RMN.
D) (R,S) n-butyl-2 cyclohexylméthyl-4 méthyl-4 imidazoline-2 one-5.
   Ce produit est obtenu sous forme d'une huile qui se prend en masse.
   Identification par IR et RMN.
   IR (chloroforme)
   1720 cm⁻¹ C=O imidazolinone
   1630 cm⁻¹ C=N
E) (R,S) n-butyl-2 cyclohexylméthyl-4 méthyl-4 [(*tert*-butoxycarbonyl-2′ biphényl-4-yl) méthyl]-1 imidazoline-2 one-5.
   Ce produit est obtenu par traitement du composé de l'étape D) avec le bromométhyl-4 *tert*-butoxycarbonyl-2′ biphényle en présence d'hydrure de sodium. Après purification par chromatographie, le produit est sous forme d'huile qui cristallise au réfrigérateur.
   Rendement : 51 %
   Fc = 73-75°C
   IR (KBr) :
   1700-1730 cm⁻¹ C=O imidazolinone et ester
   1630 cm⁻¹ C=N
F) Trifluoroacétate de (R,S) n-butyl-2 [(carboxy-2′ biphényl-4-yl) méthyl]-1 cyclohexylméthyl-4 méthyl-4 imidazoline-2 one-5.
   Rendement : 90%
   Fc : 143-146°C
   RMN :
   7,20-7,80 ppm : 8 H : aromatiques
   4,85 ppm : système AB : 2 H : N-CH₂-C₆H₄-
   2,70 ppm : t : 3 H : -CH₂-CH₂-CH₂-CH₃
   1,80-085 ppm : m : 20 H : méthyl-4 cyclohexylméthyl-4 -CH₂-CH₂-CH₂-CH₃
   0,80 ppm : t : 3 H : CH₂-CH₂-CH₂-CH₃

### EXEMPLE 3

Trifluoroacétate de (R,S) n-butyl-2 [(carboxy-2′ biphényl-4-yl) méthyl]-1 cyclohexyl-4 éthyl-4 imidazoline-2 one-5.
A) Hémioxalate de (R,S) amino-2 cyclohexyl-2 butyronitrile.
   A une solution de 1,03 g de cyanure de sodium dans 6 ml d'eau, on ajoute successivement 1,18 g de chlorure d'ammonium, 1,5 ml d'une solution d'ammoniaque à 32% puis, en 15 minutes, 2,8 g de cyclohexyléthylcétone dans 5 ml de méthanol et on chauffe à 60°C pendant 6 heures. Le milieu réactionnel est refroidi, extrait 4 fois par du DCM puis évaporé sous vide. Le résidu est traité à nouveau par les mêmes quantités de cyanure, de chlorure d'ammonium, d'ammoniaque, d'eau et de méthanol à 60°C pendant 6 heures. Le milieu réactionnel est ensuite refroidi, extrait 4 fois par du DCM, séché puis évaporé sous vide. Le résidu est repris dans 30 ml d'acétone et l'on ajoute goutte à goutte 1,1 g de dihydrate d'acide oxalique dissous dans 10 ml d'acétone. Après 15 minutes, le précipité formé est filtré, lavé à l'acétone puis à l'éther et séché sous vide. On obtient 2,87 g de produit qui devient pâteux à 120°C.
   Identification par IR et RMN.
   IR de la base libre :
   2220 cm⁻¹ C_{≡}N
B) (R,S) amino-2 cyclohexyl-2 butyramide.
   2,84 g du nitrile obtenu à l'étape A) sont ajoutés en 30 minutes à 6 ml d'acide sulfurique pur. On chauffe 1 heure à 85°C puis 30 minutes à 100°C. Après refroidissement, le milieu réactionnel est ajouté goutte à goutte à 20 ml d'ammoniaque à 32% glacée. On extrait au chloroforme puis on sèche sur sulfate de sodium et évapore sous vide. On obtient 2,5 g du produit attendu sous forme de cire.
   Identification par IR et RMN.
C) (R,S) n-butyl-2 cyclohexyl-4 éthyl-4 imidazoline-2 one-5.
   On dissout 2,45 g du produit obtenu à l'étape précédente dans 30 ml de THF : on ajoute 1,84 ml de triéthylamine puis, en 25 minutes, 1,73 ml de chlorure de valéryle dissous dans 10 ml de THF. Après 2 heures d'agitation, on ajoute 3,57 g de potasse en pastilles, puis 4 ml d'eau, puis 10 ml de méthanol et on porte au reflux pendant 3 heures. Après refroidissement, on ajoute 6 g de chlorure d'ammonium, on concentre de moitié le milieu réactionnel puis on extrait par de l'acétate d'éthyle. La phase organique est lavée par une solution saturée de chlorure de sodium, séchée sur sulfate de sodium puis concentrée sous vide. Le résidu est repris par 10 ml d'hexane et on laisse 4 heures à 0°C. Le solide formé est filtré et séché.
   m = 2,62 g
   Fc = 80-85°C
   Identification par RMN et IR (chloroforme et KBr).
D) (R,S) n-butyl-2 [(*tert*-butoxycarbonyl-2' biphényl-4-yl) méthyl]-1 cyclohexyl-4 éthyl-4 imidazoline-2 one-5.
   On opère selon le mode opératoire décrit à l'exemple 1, étape G) et l'on obtient, après chromatographie, 1,50 g du produit attendu en traitant 1 g du produit de l'étape C) par le bromométhyl-4 *tert*-butoxycarbonyl-2′ biphényle.
   IR (chloroforme)
   1700-1720 cm⁻¹ C=O imidazolinone et ester
   1635 cm⁻¹ C=N
E) Trifluoroacétate de (R,S) n-butyl-2 [(carboxy-2′ biphényl-4-yl) méthyl]-1 cyclohexyll-4 éthyl-4 imidazoline-2 one-5.

En traitant le composé obtenu l'étape précédente selon le procédé décrit à l'exemple 1, étape H), on obtient le produit attendu.
m = 1,08 g
Rendement : 85%
Fc = 159-161°C
RMN:
7,10-7,70 ppm : m : 8 H : aromatiques
4,75 ppm : s : 2 H : -N-CH₂-C₆H₄-
2,60 ppm : t : 2 H : -CH₂-CH₂-CH₂-CH₃
0,90-1,90 ppm : m : 17 H : cyclohexyle+ -CH₂-CH₃- + -CH₂-CH₂-CH₂-CH₃
0,80 ppm : t : 3 H : -CH₃
0,60 ppm : t : 3 H : -CH₃

### EXEMPLE 4

(R,S) n-butyl-2 cyclohexyl-4 méthyl-4 [((tétrazol-5-yl)-2′ biphényl-4-yl) méthyl]-1 imidazoline-2 one-5.
A) méthyl-4 (tétrazol-5-yl)-2′ biphényle.
   Le cyano-2′ méthyl-4 biphényle est préparé selon la demande de brevet européen 324 377.
   2 g de ce composé sont placés dans un ballon en présence de 4 g d'azide de tributylétain et de 20 ml de xylène et l'on chauffe à reflux pendant 110 heures. Après retour à TA, on dilue le milieu réactionnel avec du toluène puis on extrait la phase organique 3 fois par 50 ml de soude 1N. Les phases aqueuses sont réunies et lavées avec de l'éther puis on les refroidit par un bain d'eau glacée et on acidifie jusqu'à pH1-2 par addition d'acide chlorhydrique concentré. Le précipité formé est filtré, lavé à l'eau, séché sous vide, sur anhydride phosphorique. On obtient 2,18 g du produit attendu.
   Fc = 146-148°C après recristallisation dans l'acétate d'éthyle.
B) méthyl-4 (triphénylméthyltétrazol-5-yl)-2′ biphényle.
   Dans un ballon, on mélange 5,46 g du composé obtenu à l'étape A), 6,9 g de chlorure de trityle, 100 ml de DCM et 4 ml de triéthylamine. On porte à reflux pendant 4 heures puis on évapore le milieu. On reprend par de l'acétate d'éthyle, lave par de l'eau, une solution d'hydrogénosulfate de potassium à 3%, de la soude 1N, de l'eau puis une solution saturée de chlorure de sodium. On sèche sur sulfate de sodium, filtre et évapore. On obtient 11 g du produit attendu.
   Fc = 161-164°C
C) bromométhyl-4 (triphénylméthyltétrazol-5-yl)-2′ biphényle.
   On chauffe à reflux pendant 3 heures un mélange contenant 11 g du composé préparé à l'étape B), 140 ml de tétrachlorure de carbone, 4,12 g de NBS et 0,4 g de peroxyde de benzoyle. Après retour à TA, on filtre puis on évapore le filtrat. Le résidu est repris par 30 ml d'éther isopropylique. On filtre le précipité formé puis on le sèche sous vide.
   Le produit obtenu est utilisé tel quel à l'étape suivante.
D) (R,S) n-butyl-2 cyclohexyl-4 méthyl-4 [((triphénylméthyl-tétrazol-5-yl)-2′ biphényl-4-yl) méthyl]-1 imidazoline-2 one-5.
   On met en suspension sous azote, 394 mg d'hydrure de sodium à 80% dans l'huile dans 100 ml de DMF anhydre et l'on ajoute peu à peu sous agitation 1,71 g de n-butyl-2 cyclohexyl-4 méthyl-4 imidazoline-2-one-5 dans 10 ml de DMF anhydre, préparé à l'exemple 1, étape E) et on laisse 30 minutes sous agitation. On ajoute 4,86 g du composé préparé à l'étape C) et laisse 3 heures sous agitation à TA. On évapore à sec puis on reprend le résidu par 60 ml d'acétate d'éthyle, essore le milieu et évapore à sec. L'huile obtenue est chromatographiée sur silice en éluant par le mélange acétate d'éthyle/hexane (1/3 ; v/v). Après évaporation des solvants, on obtient 3,45 g du produit attendu sous forme d'une mousse solide.
   RMN :
   0,9 ppm : m : 3 H : -CH₂-CH₂-CH₂-CH₃
   1-1,8 ppm : m : 18 H : méthyl-4 +cyclohexyl-4 + -CH₂-CH₂-CH₂-CH₃
   2,35 ppm : t déformé : 2 H : -CH₂-CH₂-CH₂-CH₃
   4,5 ppm : s : 2 H : -N-CH₂-C₆H₄-
   6,70-8 ppm : m : 23 H : aromatiques
E) (R,S) n-butyl-2 cyclohexyl-4 méthyl-4 [((tétrazol-5-yl)-2′ biphényl-4-yl) méthyl]-1 imidazoline-2 one-5.

On met en solution 3,38 g du composé préparé à l'étape D) dans un mélange de 40 ml de méthanol et 20 ml de THF. On ajoute 3,5 ml d'acide chlorhydrique 4N et laisse 3 heures sous agitation à TA. Après évaporation à sec, on reprend le résidu par 10 ml de soude 2N et 10 ml d'éther puis on agite jusqu'à dissolution. On extrait 2 fois par de l'acide chlorhydrique dilué puis on extrait 3 fois à l'acétate d'éthyle ; on sèche sur sulfate de sodium et on évapore à sec. On obtient 1,72 g du produit attendu sous forme d'une mousse solide blanche.
RMN :
0,9 ppm : m : 3 H : -CH₂-CH₂-CH₂-CH₃
0,95-2,7 ppm : m : 18 H : cyclohexyl-4 + méthyl-4 +-CH₂-CH₂-CH₂-CH₃
2,1 ppm : t : 2 H : -CH₂-CH₂-CH₂-CH₃
4,4-4,7 ppm : système AB : 2 H : -N-CH₂-C₆H₄-
6,95-7,1 ppm : q : 4 H : aromatiques
7,3-7,6 ppm : m : 3 H : aromatiques
7,8 ppm : d : 1 H : aromatiques

### EXEMPLE 5

n-butyl-2 cyclohexyl-4 méthyl-4 [((tétrazol-5-yl)-2′ biphényl-4-yl) méthyl]-1 imidazoline-2 one-5, lévogyre.
A) méthyl-5 phényl-5 hydantoïne.
   30 g d'acétophénone, dilués dans 250 ml d'alcool 95, sont ajoutés en 30 minutes à un mélange de 18,35 g de cyanure de sodium et 125 g de carbonate d'ammonium dans 250 ml d'eau et l'on chauffe à 60-65°C, sous agitation, pendant 22 heures. Le milieu réactionnel est concentré sous vide de moitié et le solide qui a précipité est filtré, lavé à l'eau, à l'éther, puis séché sous vide. On obtient 38 g de solide blanc, identifié par IR.
   Fc = 190-192°C
B) Acide (R,S) amino-2 phényl-2 propionique.
   20 g du composé préparé à l'étape précédente sont ajoutés à un mélange de 75 g d'octahydrate d'hydroxyde de baryum dans 500 ml d'eau, puis on chauffe à 160°C pendant 5 heures dans un tube d'acier. Le milieu réactionnel est saturé de carboglace, puis le précipité est filtré. Le filtrat est concentré sous vide, le solide blanc formé est repris dans de l'acétone, filtré, lavé à l'acétone, à l'éther, puis séché. On obtient 15.3 g de l'acide attendu.
   Fc = 260-265°C (avec décomposition).
C) Ester éthylique de l'acide (R,S) amino-2 phényl-2 propionique.
   A une solution de 80 g de gaz chlorhydrique dans 210 ml d'éthanol absolu, on ajoute à la spatule, sous agitation, 24 g d'acide préparé à l'étape précédente et l'on chauffe à reflux pendant 6 heures et demie. On concentre sous vide, on reprend le résidu dans 600 ml d'acétate d'éthyle et 100 ml d'eau et l'on ajoute de la soude 2N jusqu'à pH9. La phase organique est décantée, lavée à l'eau par une solution saturée de chlorure de sodium, séchée sur sulfate de sodium puis concentrée sous vide. On obtient 25,5 g du produit attendu sous forme d'huile. Identification par IR.
D) Ester éthylique dextrogyre de l'acide amino-2 phényl-2 propionique.
   On sépare les énantiomères de l'ester préparé à l'étape précédente par la méthode décrite par Y. Sugi et S. Mitsui dans Bull. Chem. Soc. Japan, 1969, 42, 2984-2988. Aux 25,5 g d'ester obtenus à l'étape C), dilués dans 210 ml d'éthanol absolu, on ajoute 19,8 g d'acide (L) (+) tartrique. On chauffe à 60°C pour tout dissoudre puis on laisse à TA pendant 4 heures. Les cristaux formés sont filtrés puis rincés par 2 fois 70 ml d'alcool absolu, puis ils sont redissous dans 200 ml d'alcool à ébullition puis on laisse à TA pendant 72 heures. Les cristaux en aiguille formés sont filtrés, rincés 2 fois par 30 ml d'alcool puis séchés sous vide. On obtient 11,9 g du sel d'acide tartrique de l'ester dextrogyre attendu.
   Fc = 172-173°C
   [α]_{D} = + 44,5° (C=1, eau)
   La solution alcoolique restante est enrichie en sel d'acide tartrique de l'ester lévogyre.
   6,1 g du sel d'acide tartrique de l'ester dextrogyre sont repris dans 30 ml d'eau puis 200 ml d'acétate d'éthyle et l'on ajoute de la soude 5N jusqu'à pH9. La phase organique est décantée, lavée à l'eau, par une solution saturée de chlorure de sodium, séchée sur sulfate de sodium puis concentrée sous vide. On obtient 3,33 g du produit attendu sous forme d'huile.
   [α]_{D} = + 24° (C=2, éthanol)
   Identification par RMN.
E) ester éthylique dextrogyre de l'acide amino-2 cyclohexyl-2 propionique.
   3,30 g de l'ester dextrogyre, obtenu à l'étape précédente, sont dilués dans 120 ml d'acide acétique ; on ajoute 1,5 g d'oxyde de platine puis on hydrogène à pression atmosphérique. Après 40 heures d'hydrogénation, le milieu réactionnel est filtré puis concentré sous vide. Le résidu est repris dans un mélange éther/eau et l'on ajoute de l'acide chlorhydrique 6N, jusqu'à pH2. La phase aqueuse est décantée. On ajoute de l'acétate d'éthyle puis de la soude 5N, jusqu'à pH 9,5. La phase organique est décantée, lavée à l'eau, par une solution de chlorure de sodium saturée, séchée sur sulfate de sodium, puis concentrée sous vide. On obtient 3,10 g du produit attendu.
   [α]_{D} = +18° (C=2, éthanol). Littérature : W.A. Bonner et al., J Amer. Chem. Soc., 1956, 78, 3218-3221.
   Identification par RMN.
F) n-butyl-2 cyclohexyl-4 méthyl-4 imidazoline-2 one-5, lévogyre.
   On porte à reflux sous agitation un mélange contenant 3 g d'ester dextrogyre obtenu à l'étape précédente, 4,7 g de valérimidate d'éthyle et 8 gouttes d'acide acétique dans 15 ml de xylène.
   Après 7 heures de reflux, le milieu réactionnel est concentré sous vide. Le résidu est chromatographié sur silice en éluant par un mélange chloroforme/méthanol/acide acétique (95/9/3) ; les fractions contenant le produit sont rassemblées et concentrées sous vide. Le résidu est repris dans un mélange acétate d'éthyle/eau et on amène à pH 9 par addition de soude 5N. La phase organique est décantée, lavée à l'eau, par une solution saturée de chlorure de sodium, séchée sur sulfate de sodium et concentrée sous vide. On obtient une huile qui se transforme en solide amorphe.
   m = 2,36 g
   [α]_{D} = -57,2° (c=1, chloroforme)
   IR (chloroforme)
   1720 cm⁻¹ C=O
   1640 cm⁻¹ C=N
   Le spectre IR confirme la forme one-5 de l'imidazolinone en solution.
G) n-butyl-2 cyclohexyl-4 méthyl-4 [((triphénylméthyltétrazol-5-yl)-2′ biphényl-4-yl) méthyl]-1 imidazoline-2 one-5, lévogyre.
   Ce composé est préparé à partir du produit préparé à l'étape F) en suivant le mode opératoire décrit à l'exemple 4, étape D).
   Rendement : 73%
   [α]_{D} = -22,8° (c=1, chloroforme)
   RMN : superposable à celle du composé de l'exemple 4, étape D).
H) n-butyl-2 cyclohexyl-4 méthyl-4 [((tétrazol-5-yl)-2′ biphényl-4-yl) méthyl]-1 imidazoline-2 one-5, lévogyre.

Ce composé est préparé à partir du produit préparé à l'étape G en suivant le mode opératoire décrit à l'exemple 4, étape E).
Rendement : 85%
[α]_{D} = -25,9° (c= 1, méthanol)
RMN : superposable à celle du composé de l'exemple 4, étape E).

### EXEMPLE 6

n-butyl-2 cyclohexyl-4 méthyl-4 [((tétrazol-5-yl)-2′ biphényl-4-yl) méthyl]-1 imidazoline-2 one-5, dextrogyre.
A) ester éthylique lévogyre de l'acide amino-2 phényl-2 propionique.
   On concentre sous vide la solution alcoolique obtenue à l'exemple 5, étape D), après la séparation des cristaux de sel d'acide tartrique de l'ester éthylique dextrogyre de l'acide amino-2 phényl-2 propionique. Le résidu solide est repris dans 150 ml d'eau et 600 ml d'acétate d'éthyle et on amène à pH 9 par addition de soude 5N. La phase organique est décantée, lavée à l'eau, par une solution saturée de chlorure de sodium, séchée sur sulfate de sodium puis concentrée sous vide. On obtient 20,6 g d'ester enrichi en levogyre.
   A 20,5 g de cet ester, dilués dans 200 ml d'éthanol absolu, on ajoute 15,9 g d'acide D(-) tartrique et on dissout à ébullition de l'alcool. Après 5 heures à TA, les cristaux en aiguille formés sont filtrés, lavés 2 fois par 50 ml d'alcool absolu puis séchés sous vide.
   On obtient 16,3 g de sel tartrique du produit attendu.
   Fc = 172-173°C
   [α]_{D} = - 45,2° (C=1, eau)
   6 g du sel obtenu sont repris dans 50 ml d'eau et 200 ml d'acétate d'éthyle et on amène à pH9,5 par addition de soude 5N. La phase organique est décantée, lavée à l'eau, par une solution saturée de chlorure de sodium, puis séchée sur sulfate de sodium et concentrée sous vide. On obtient 3,31 g du produit attendu sous forme d'huile; identifiée par RMN.
   [α]_{D} = - 25,5° (C=2, éthanol)
B) ester éthylique lévogyre de l'acide amino-2 cyclohexyl-2 propionique.
   On opère comme à l'exemple 5, étape E) et l'on obtient 3,20 g du produit attendu à partir de 3,30 g du composé de l'étape A).
   [α]_{D} = - 19,2° (C=1, éthanol)
C) n-butyl-2 cyclohexyl-4 méthyl-4 imidazoline-2 one-5, dextrogyre.
   On opère comme à l'exemple 5, étape F).
   [α]_{D} = + 56,9° (c=1, chloroforme)
   Les spectres RMN et IR sont superposables à ceux de l'isomère lévogyre, préparé à l'exemple 5, étape F).
D) n-butyl-2 cyclohexyl-4 méthyl-4 [(triphénylméthyltétrazol-5-yl)-2′ biphényl-4-yl) méthyl]-1 imidazoline-2 one-5, dextrogyre.
   On opère selon le mode opératoire décrit à l'exemple 5, étape G) et l'on obtient 2,3 g du produit attendu sous forme d'un solide blanc, à partir de 1,1 g du composé préparé à l'étape C).
   [α]_{D} = -23,8° (c=1, méthanol) et spectre RMN superposable à celui du composé de l'exemple 4, étape D).
E) n-butyl-2 cyclohexyl-4 méthyl-4 [((tétrazol-5-yl)-2′ biphényl-4-yl) méthyl]-1 imidazoline-2 one-5, dextrogyre.

On opère selon le mode opératoire décrit à l'exemple 5, étape H) et l'on obtient 1,1 g du produit attendu sous forme d'un solide blanc, à partir de 2,15 g du composé préparé à l'étape D).
[α]_{D} = +27,1° (c=1, méthanol)

Le spectre RMN est superposable à celui du composé de l'exemple 4.

De la même façon, en suivant le mode opératoire décrit à l'exemple 3, on a préparé les composés suivants selon l'invention :

### EXEMPLE 7

Trifluoroacétate de (R,S) n-butyl-2 [(carboxy-2' biphényl-4-yl) méthyl]-1 cyclopropyl-4 éthyl-4 imidazoline-2 one-5.
Fc = 149-150°C
RMN :
7,05-7,80 ppm : m : 8 H : aromatiques
4,7 ppm : s : 2 H : -N-CH₂-C₆H₄-
2,45 ppm : t : 2 H : -CH₂-CH₂-CH₂-CH₃
1,05-1,45 ppm : m+s : 8 H : -CH₂-CH₂-CH₂-CH₃ + CH cyclopropane + CH₃ en 4
0,70 ppm : t : 3 H : CH₃-(CH₂)₃-
0,05-0,45 ppm : m : 4 H : 2CH₂ cyclopropane

### EXEMPLE 8

Trifluoroacétate de (R,S) n-butyl-2 [(carboxy-2′ biphényl-4-yl) méthyl]-1 dicyclopropyl-4,4 imidazoline-2 one-5.
Fc = 132-134°C
Spectre de masse : MH+ : 431
RMN :
7,15-7,80 ppm : m : 8 H : aromatiques
4,75ppm : s : 2 H : -N-CH₂-C₆H₄-
2,50 ppm : t : 2 H : -CH₂-CH₂-CH₂-CH₃
1,1-1,60 ppm : m : 6 H : -CH₂-CH₂-CH₂-CH₃ + 2 CH cyclopropane
0,80 ppm : t : 3 H : (CH₂)₃-CH₃
0,10-0,80 ppm : m : 8 H : 4 CH₂ cyclopropane

### EXEMPLE 9

Trifluoroacétate de (R,S) n-butyl-2 [(carboxy-2′ biphényl-4-yl) méthyl]-1 cyclopentyl-4 méthyl-4 imidazoline-2 one-5.
Fc = 104-107°C
RMN :
7,20-7,80 ppm : m : 8 H : aromatiques
4,85 ppm: système AB : 2 H : -N-CH₂-C₆H₄-
2,75 ppm : t déformé : 2 H : -CH₂-CH₂-CH₂-CH₃
2,20-1,00 ppm : m+s : 16 H : cyclopentane + CH₃- en 4 + -CH₂-CH₂-CH₃
0,80 ppm : t : 3 H : CH₃-(CH₂)₃-

### EXEMPLE 10

(R,S) n-butyl-2 cyclopentyl-4 méthyl-4 [((tétrazol-5-yl)-2′ biphényl-4-yl) méthyl]-1 imidazoline-2 one-5.

Ce composé est préparé en suivant le mode opératoire décrit à l'exemple 4.
Fc = 78-80°C
RMN : (CDCl₃)
7,05-7,8 ppm : m : 8 H : aromatiques
4,70 ppm : s : 2 H : -N-CH₂-C₆H₄-
2,40 ppm : t : 2 H : -CH₂-CH₂-CH₂-CH₃
2,15-1,20 ppm : m : 13 H : -CH₂-CH₂-CH₂-CH₃ + cyclopentyl-4
1,20 ppm : s : 3 H : CH₃-4
0,95 ppm : t : 3 H : -CH₂-CH₂-CH₂-CH₃-

## Revendications

1. Un composé de formule : 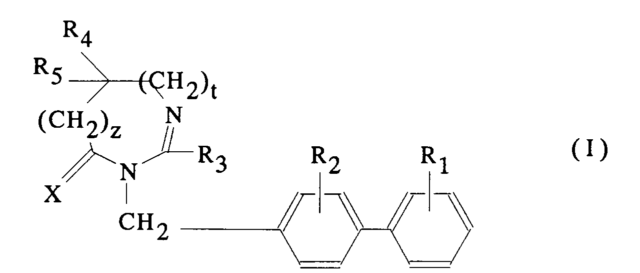 dans laquelle :
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyano-acétamide, un N-hydroxy-acétamide, un N-((carboxy-4) thiazol-1,3-yl-2 acétamide, un ureido, un cyano-2 guanidinocarbonyle, un cyano-2 guanidinométhyle, un imidazol-1-yl-carbonyle, un cyano-3 méthyl-2 isothioureidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ représente un alkyle en C₁-C₆, non substitué ou substitué par un ou plusieurs atomes d'halogène ;
- R₅ représente un cycloalkyle ou un cycloalkylméthyle, ledit cycloalkyle étant en C₃-C₇ non substitué ou substitué par un ou plusieurs atomes d'halogène ;
- ou R₄ et R₅ représentent chacun un cyclopropyle ;
- X représente un atome d'oxygène ou un atome de soufre ;
- z et t sont nuls ou l'un est nul et l'autre représente un ;
et ses sels.

2. Composé selon la revendication 1, caractérisé en ce que R₁ est en position ortho et représente un groupe carboxy ou tétrazolyle et R₂ est l'hydrogène.

3. Composé selon l'une quelconque des revendications 1 à 2, caractérisé en ce que R₄ est le méthyle et R₅ est le cyclohexyle.

4. Composé selon l'une quelconque des revendications 1 à 3, caractérisé en ce que R₃ représente un groupe alkyle droit en C₁-C₆.

5. Composé selon l'une quelconque des revendications 1 à 4, caractérisé en ce que X est l'oxygène.

6. Composé selon l'une quelconque des revendications 1 à 5, caractérisé en ce que z = t = O.

7. Composé selon la revendication 1, caractérisé en ce qu'il est le n-butyl-2 méthyl-4 cyclohexyl-4 [((tétrazol-5-yl)-2′ biphényl-4-yl) méthyl]-1 imidazoline-2 one-5 ou l'un de ses sels avec des acides ou des bases.

8. Procédé pour la préparation d'un composé (I) selon l'une quelconque des revendications 1 à 7 caractérisé, en ce que :
a1) on fait réagir un dérivé hétérocyclique de formule : 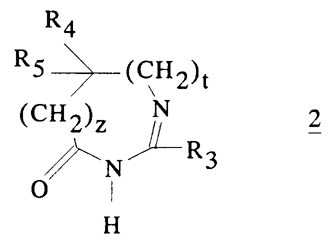 dans lequelle, X, z, t, R₃, R₄ et R₅ ont les significations indiquées ci-dessus pour (I), dans la revendication 1, sur un dérivé du (biphényl-4-yl) méthyle de formule : 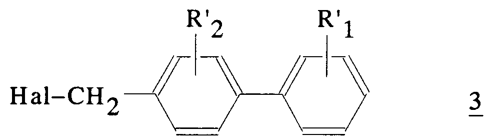 dans laquelle Hal représente un atome d'halogène et R′₁ et R′₂ représentent respectivement soit R₁ et R₂, soit un précurseur de R₁ et R₂ ;
b1) éventuellement, le composé, ainsi obtenu de formule : 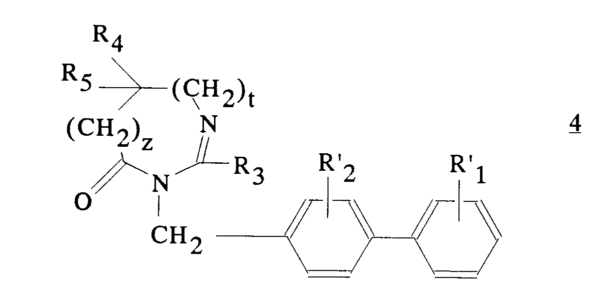 est traité par le réactif de Lawesson [bis(méthoxy-4 phényl)-2,4 dithia-1,3 diphosphétane-2,4 disulfure-2,4] ;
c1) le composé obtenu en a1) ou en b1), de formule : 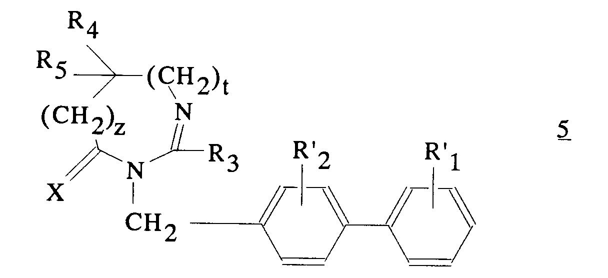 dans laquelle X représente un atome d'oxygène ou un atome de soufre, est traité pour préparer le composé (I) par transformation des groupes R′₁ et/ou R′₂ en respectivement R₁ et/ou R₂
d1) le composé ainsi obtenu est éventuellement transformé en l'un de ses sels.

9. Procédé pour la préparation d'un composé (I), selon l'une quelconque des revendications 1 à 7 , caractérisé en ce que :
a2) on fait réagir un aminoacide de formule : 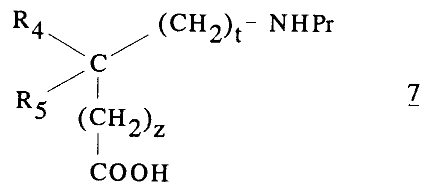 dans laquelle z, t, R₄ et R₅ ont les significations indiquées ci-dessus pour (I) et dont la fonction amine est protégée par le groupe Pr, sur un dérivé (biphényl-4-yl) méthylamine de formule : 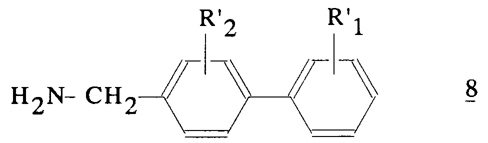 dans laquelle R′₁ et/ou R′₂ représentent respectivement soit R₁ et R₂, soit un groupement précurseur de R₁ et R₂ ;
b2) après déprotection de l'amine, le composé ainsi obtenu de formule : 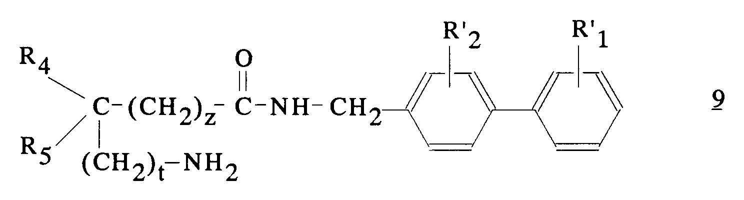 est ensuite traité par un ortho-ester d'alkyle de formule R₃C(OR)₃ (10), dans laquelle R₃ a la signification indiquée ci-dessus pour (I) dans la revendication 1 et R est un alkyle en C₁-C₄ ;
c2) éventuellement, le composé ainsi obtenu de formule : 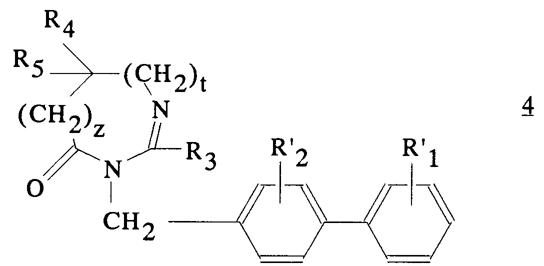 est traité par le réactif de Lawesson [bis(méthoxy-4 phényl)-2,4 dithia-1,3 diphosphénate-2,4 disulfure 2,4] ;
d2) le composé ainsi obtenu en b2) ou en c2) de formule : 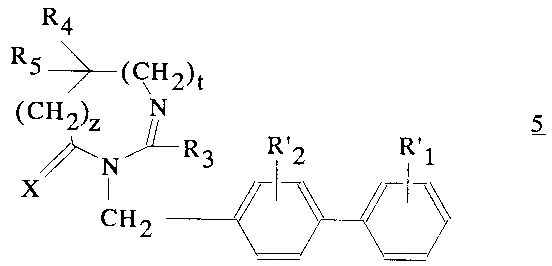 est ensuite traité dans des conditions convenables pour préparer le composé (I) par transformation des groupes R′₁ et/ou R′₂ en, respectivement, les groupes R₁ et/ou R₂ ;
e2) le composé ainsi obtenu est ensuite éventuellement transformé en l'un de ses sels.

10. Un compose de formule : 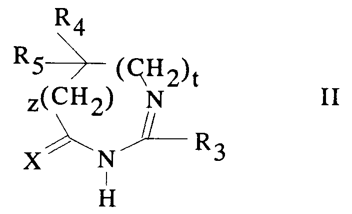 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ représente un alkyle en C₁-C₆, non substitué ou substitué par un ou plusieurs atomes d'halogène ;
- R₅ représente un cycloalkyle ou un cycloalkylméthyle, le cycloalkyle étant en C₃-C₇, non substitué ou substitué par un ou plusieurs atomes d'halogène ;
- ou encore R₄ et R₅ représentent chacun un cyclopropyle ;
- X représente un atome d'oxygène ;
- z et t sont nuls ou l'un est nul et l'autre représente un ;
avec la limitation que R₃ est autre que phényle ou phényle substitué lorsque simultanément z=t=0, R₅ représente un cycloalkyle.

11. Un composé selon la revendication 10 de formule : 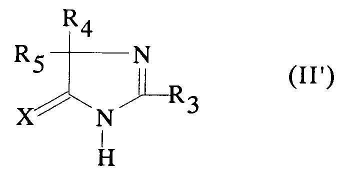 dans laquelle R₃, R₄, R₅ et X ont les définitions données ci-dessus pour (II) dans la revendication 10 et R₃ est un alkyle en C₁-C₆.

12. Un composé selon la revendication 11, de formule : 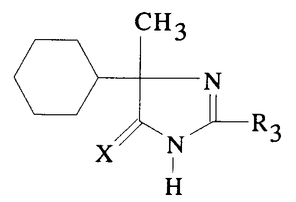 dans laquelle R₃ est un alkyle en C₁-C₆ et X est l'oxygène.

13. Un composé selon la revendication 10, de formule : 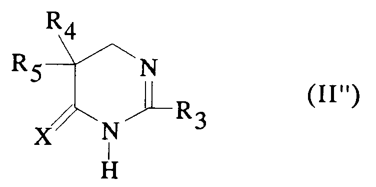 dans laquelle R₃, R₄, R₅ et X ont les définitions données ci-dessus pour (II), dans la revendication 10.

14. Un composé selon la revendication 10, de formule : 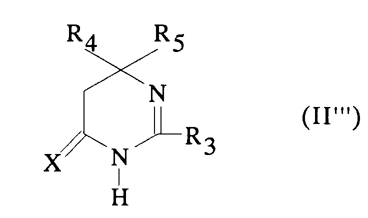 dans laquelle X, R₃, R₄ et R₅ ont les significations indiquées pour (II) dans la revendication 10.

15. Procédé pour la préparation d'un composé selon l'une quelconque des revendications 10 à 14, caractérisé en ce que on fait agir sur un composé de formule : 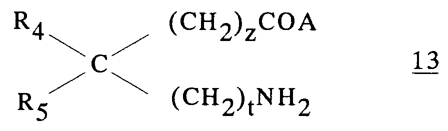 dans laquelle R₄ et R₅ ont les définitions indiquées ci-dessus pour (II) dans la revendication 10, A représente un groupe OH, un groupeNH₂ ou un groupe OR′, R′ étant l'hydrogène ou un akyle en C₁-C₄, un composé de formule :
R₃ - B 14
dans laquelle R₃ a la définition indiquée ci-dessus pour (II) dans la revendication 10 et B représente :
- un groupement C (OR)₃
- un groupement 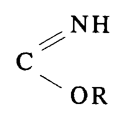 ou
- un groupement COHal ;
R étant un alkyle en C₁-C₄ et Hal désignant un atome d'halogène, de préférence le chlore.

16. Composition pharmaceutique contenant à titre de principe actif un composé selon l'une quelconque des revendication 1 à 7.

17. Composition pharmaceutique contenant un composé selon l'une quelconque des revendication 1 à 7 en association avec un composé bêtabloquant.

18. Composition pharmaceutique contenant un composé selon l'une quelconque des revendication 1 à 7 en association avec un diurétique.

19. Composition pharmaceutique contenant un composé selon l'une quelconque des revendication 1 à 7 en association avec un antiinflammatoire non stéroïdien.

20. Composition pharmaceutique contenant un composé selon l'une quelconque des revendication 1 à 7 en association avec un antagoniste calcique.

21. Composition pharmaceutique contenant un composé selon l'une quelconque des revendication 1 à 7 en association avec un tranquillisant.

## Revendications (Revendications pour l'(les) Etat(s) contractant(s) suivant(s): GR)

1. Un composé de formule : 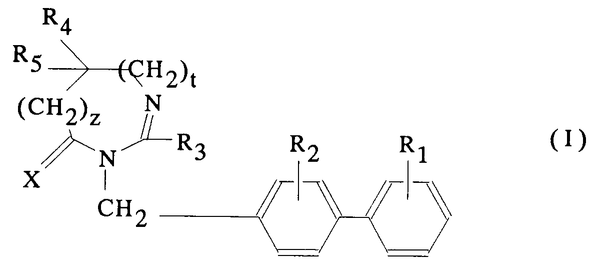 dans laquelle :
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyano-acétamide, un N-hydroxy-acétamide, un N-((carboxy-4) thiazol- 1,3-yl-2 acétamide, un ureido, un cyano-2 guanidinocarbonyle, un cyano-2 guanidinométhyle, un imidazol-1-yl-carbonyle, un cyano-3 méthyl-2 isothioureidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ représente un alkyle en C₁-C₆, non substitué ou substitué par un ou plusieurs atomes d'halogène ;
- R₅ représente un cycloalkyle ou un cycloalkylméthyle, ledit cycloalkyle étant en C₃-C₇ non substitué ou substitué par un ou plusieurs atomes d'halogène ;
- ou R₄ et R₅ représentent chacun un cyclopropyle ;
- X représente un atome d'oxygène ou un atome de soufre ;
- z et t sont nuls ou l'un est nul et l'autre représente un ;
et ses sels.

2. Composé selon la revendication 1, caractérisé en ce que R₁ est en position ortho et représente un groupe carboxy ou tétrazolyle et R₂ est l'hydrogène.

3. Composé selon l'une quelconque des revendications 1 à 2, caractérisé en ce que R₄ est le méthyle et R₅ est le cyclohexyle.

4. Composé selon l'une quelconque des revendications 1 à 3, caractérisé en ce que R₃ représente un groupe alkyle droit en C₁-C₆.

5. Composé selon l'une quelconque des revendications 1 à 4, caractérisé en ce que X est l'oxygène.

6. Composé selon l'une quelconque des revendications 1 à 5, caractérisé en ce que z = t = O.

7. Composé selon la revendication 1, caractérisé en ce qu'il est le n-butyl-2 méthyl-4 cyclohexyl-4 [((tétrazol-5-yl)-2′ biphényl-4-yl) méthyl]-1 imidazoline-2 one-5 ou l'un de ses sels avec des acides ou des bases.

8. Procédé pour la préparation d'un composé (I) selon l'une quelconque des revendications 1 à 7 caractérisé, en ce que :
a1) on fait réagir un dérivé hétérocyclique de formule : 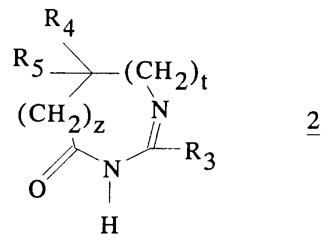 dans lequelle, X, z, t, R₃, R₄ et R₅ ont les significations indiquées ci-dessus pour (I), dans la revendication 1, sur un dérivé du (biphényl-4-yl) méthyle de formule : 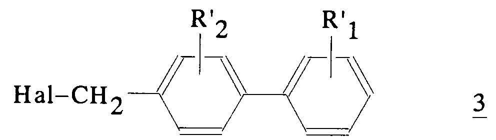 dans laquelle Hal représente un atome d'halogène et R′₁ et R′₂ représentent respectivement soit R₁ et R₂, soit un précurseur de R₁ et R₂ ;
b1) éventuellement, le composé, ainsi obtenu de formule : 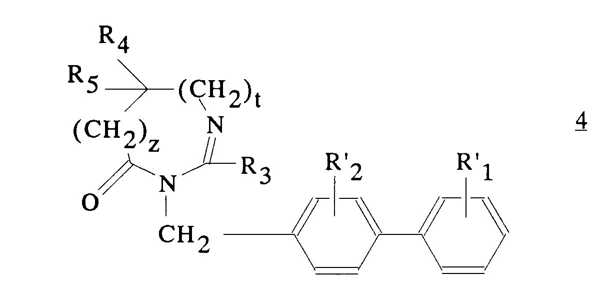 est traité par le réactif de Lawesson [bis(méthoxy-4 phényl)-2,4 dithia-1,3 diphosphétane-2,4 disulfure-2,4] ;
c1) le composé obtenu en a1) ou en b1), de formule : 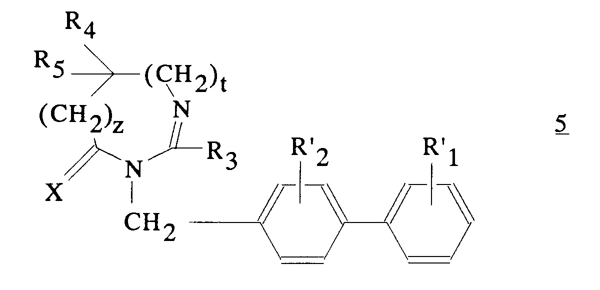 dans laquelle X représente un atome d'oxygène ou un atome de soufre, est traité pour préparer le composé (I) par transformation des groupes R′₁ et/ou R′₂ en respectivement R₁ et/ou R₂
d1) le composé ainsi obtenu est éventuellement transformé en l'un de ses sels.

9. Procédé pour la préparation d'un composé (I), selon l'une quelconque des revendications 1 à 7 , caractérisé en ce que :
a2) on fait réagir un aminoacide de formule : 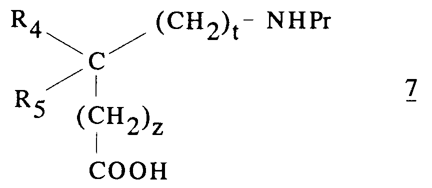 dans laquelle z, t, R₄ et R₅ ont les significations indiquées ci-dessus pour (I) et dont la fonction amine est protégée par le groupe Pr, sur un dérivé (biphényl-4-yl) méthylamine de formule : 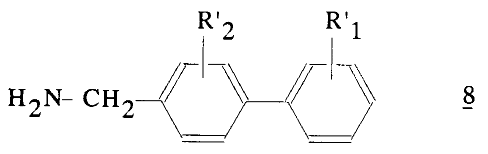 dans laquelle R′₁ et/ou R′₂ représentent respectivement soit R₁ et R₂, soit un groupement précurseur de R₁ et R₂ ;
b2) après déprotection de l'amine, le composé ainsi obtenu de formule : 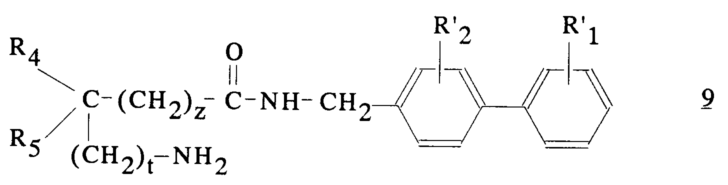 est ensuite traité par un ortho-ester d'alkyle de formule R₃C(OR)₃ (10), dans laquelle R₃ a la signification indiquée ci-dessus pour (I) dans la revendication 1 et R est un alkyle en C₁-C₄ ;
c2) éventuellement, le composé ainsi obtenu de formule : 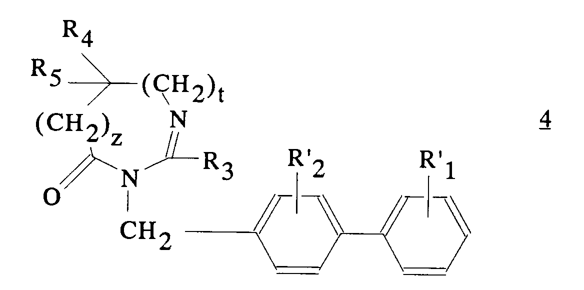 est traité par le réactif de Lawesson [bis(méthoxy-4 phényl)-2,4 dithia-1,3 diphosphénate-2,4 disulfure 2,4] ;
d2) le composé ainsi obtenu en b2) ou en c2) de formule : 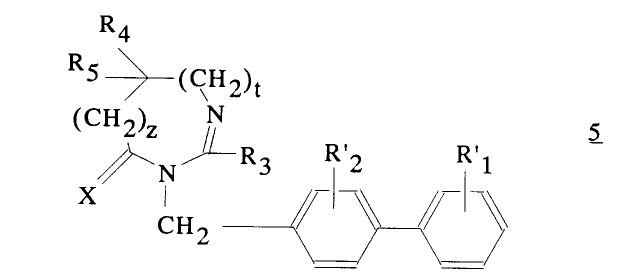 est ensuite traité dans des conditions convenables pour préparer le composé (I) par transformation des groupes R′₁ et/ou R′₂ en, respectivement, les groupes R₁ et/ou R₂ ;
e2) le composé ainsi obtenu est ensuite éventuellement transformé en l'un de ses sels.

10. Un compose de formule : 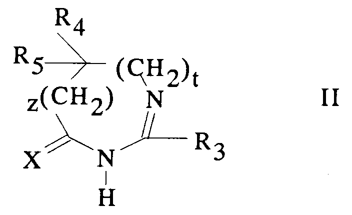 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ représente un alkyle en C₁-C₆, non substitué ou substitué par un ou plusieurs atomes d'halogène ;
- R₅ représente un cycloalkyle ou un cycloalkylméthyle, le cycloalkyle étant en C₃-C₇, non substitué ou substitué par un ou plusieurs atomes d'halogène ;
- ou encore R₄ et R₅ représentent chacun un cyclopropyle ;
- X représente un atome d'oxygène ;
- z et t sont nuls ou l'un est nul et l'autre représente un ;
avec la limitation que R₃ est autre que phényle ou phényle substitué lorsque simultanément z=t=0, R₅ représente un cycloalkyle.

11. Un composé selon la revendication 10 de formule : 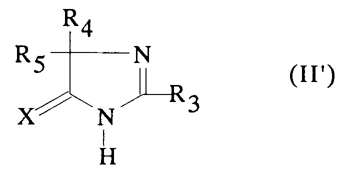 dans laquelle R₃, R₄, R₅ et X ont les définitions données ci-dessus pour (II) dans la revendication 10 et R₃ est un alkyle en C₁-C₆.

12. Un composé selon la revendication 11, de formule : 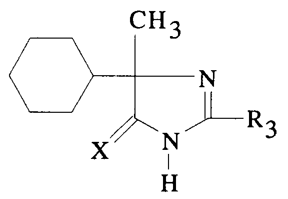 dans laquelle R₃ est un alkyle en C₁-C₆ et X est l'oxygène.

13. Un composé selon la revendication 10, de formule : 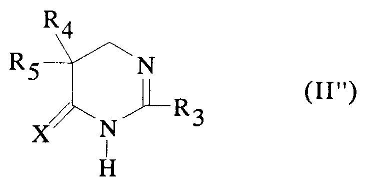 dans laquelle R₃, R₄, R₅ et X ont les définitions données ci-dessus pour (II), dans la revendication 10.

14. Un composé selon la revendication 10, de formule : 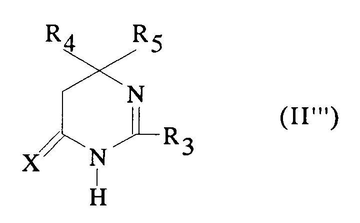 dans laquelle X, R₃, R₄ et R₅ ont les significations indiquées pour (II) dans la revendication 10.

15. Procédé pour la préparation d'un composé selon l'une quelconque des revendications 10 à 14, caractérisé en ce que on fait agir sur un composé de formule : 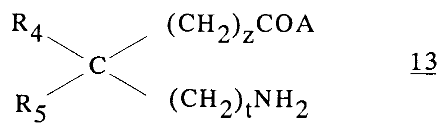 dans laquelle R₄ et R₅ ont les définitions indiquées ci-dessus pour (II) dans la revendication 10, A représente un groupe OH, un groupeNH₂ ou un groupe OR′, R′ étant l'hydrogène ou un akyle en C₁-C₄, un composé de formule :
R₃ - B 14
dans laquelle R₃ a la définition indiquée ci-dessus pour (II) dans la revendication 10 et B représente :
- un groupement C (OR)₃
- un groupement 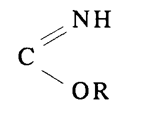 ou
- un groupement CO Hal ;
R étant un alkyle en C₁-C₄ et Hal désignant un atome d'halogène, de préférence le chlore.

16. Procédé pour la préparation d'une compositoon pharmaceutique, caractérisé en ce qu'il consiste à mélanger un composé selon l'une quelconque des revendications 1 à 7 avec un véhicule pharmaceutiquement acceptable.

17. Procédé pour la préparation d'une composition pharmaceutique, caractérisé en ce qu'il consiste à associer un composé selon l'une quelconque des revendications 1 à 7 avec un composé bêtabloquant.

18. Procédé pour la préparation d'une composition pharmaceutique, caractérisé en ce qu'il consiste à associer un composé selon l'une quelconque des revendications 1 à 7 avec un diurétique.

19. Procédé pour la préparation d'une composition pharmaceutique, caractérisé en ce qu'il consiste à associer un composé selon l'une quelconque des revendications 1 à 7 avec un anti-inflammatoire non stéroïdien.

20. Procédé pour la préparation d'une composition pharmaceutique, caractérisé en ce qu'il consiste à associer un composé selon l'une quelconque des revendications 1 à 7 avec un antagoniste calcique.

21. Procédé pour la préparation d'une composition pharmaceutique, caractérisé en ce qu'il consiste à associer un composé selon l'une quelconque des revendications 1 à 7 avec un tranquilisant.

## Revendications (Revendications pour l'(les) Etat(s) contractant(s) suivant(s): ES)

1. Procédé pour la préparation d'un composé de formule : 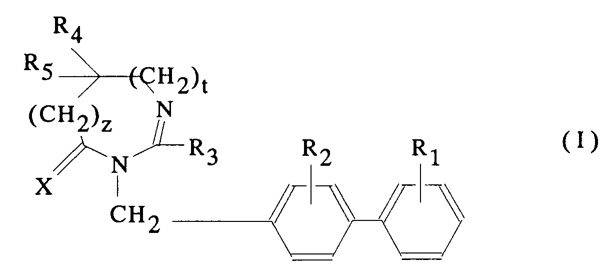 dans laquelle :
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyano-acétamide, un N-hydroxy-acétamide, un N-((carboxy-4) thiazol-1,3-yl-2 acétamide, un ureido, un cyano-2 guanidinocarbonyle, un cyano-2 guanidinométhyle, un imidazol-1-yl-carbonyle, un cyano-3 méthyl-2 isothioureidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ représente un alkyle en C₁-C₆, non substitué ou substitué par un ou plusieurs atomes d'halogène ;
- R₅ représente un cycloalkyle ou un cycloalkylméthyle, ledit cycloalkyle étant en C₃-C₇ non substitué ou substitué par un ou plusieurs atomes d'halogène ;
- ou R₄ et R₅ représentent chacun un cyclopropyle ;
- X représente un atome d'oxygène ou un atome de soufre ;
- z et t sont nuls ou l'un est nul et l'autre représente un ;
et ses sels, caractérisé en ce que :
a1) on fait réagir un dérivé hétérocyclique de formule : 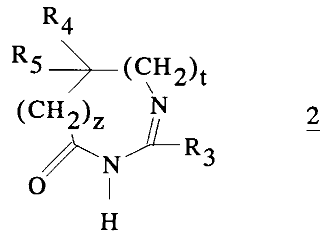 dans laquelle, z, t, R₃, R₄ et R₅ ont les significations indiquées ci-dessus pour (I), sur un dérivé du (biphényl-4-yl) méthyle de formule : 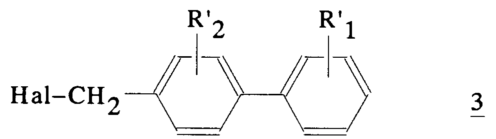 dans laquelle Hal représente un atome d'halogène et R′₁ et R′₂ représentent respectivement soit R₁ et R₂, soit un précurseur de R₁ et R₂ ;
b1) éventuellement, le composé, ainsi obtenu de formule : 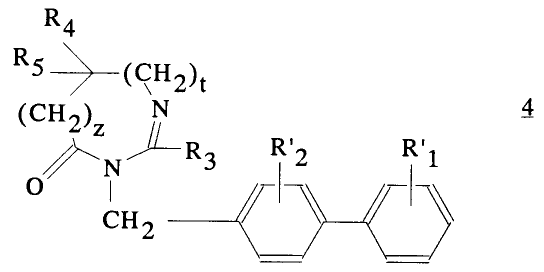 est traité par le réactif de Lawesson [bis(méthoxy-4 phényl)-2,4 dithia-1,3 diphosphétane-2,4 disulfure-2,4] ;
c1) le composé obtenu en a1) ou en b1), de formule : 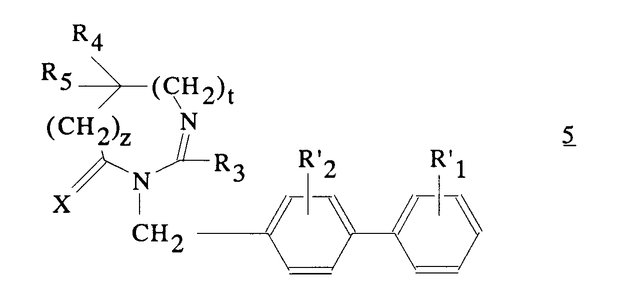 dans laquelle X représente un atome d'oxygène ou un atome de soufre, est traité pour préparer le composé (I) par transformation des groupes R′₁ et/ou R′₂ en respectivement R₁ et/ou R₂
d1) le composé ainsi obtenu est éventuellement transformé en l'un de ses sels.

2. Procédé pour la préparation d'un composé de formule I et ses sels tel que défini dans la revendication 1, caractérisé en ce que :
a2) on fait réagir un aminoacide de formule : 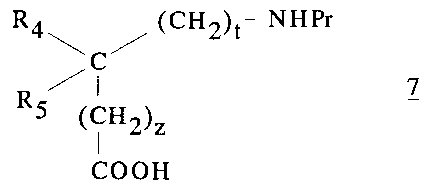 dans laquelle z, t, R₄ et R₅ ont les significations indiquées ci-dessus pour (I) et dont la fonction amine est protégée par le groupe Pr, sur un dérivé (biphényl-4-yl) méthylamine de formule : 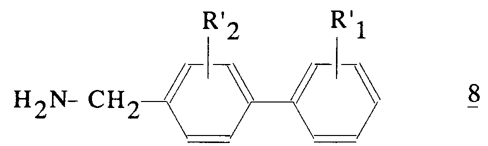 dans laquelle R′₁ et/ou R′₂ représentent respectivement soit R₁ et R₂, soit un groupement précurseur de R₁ et R₂ ;
b2) après déprotection de l'amine, le composé ainsi obtenu de formule : 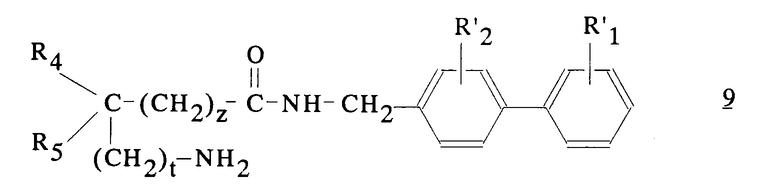 est ensuite traité par un ortho-ester d'alkyle de formule R₃C(OR)₃ (10), dans laquelle R₃ a la signification indiquée ci-dessus pour (I) dans la revendication 1 et R est un alkyle en C₁-C₄ ;
c2) éventuellement, le composé ainsi obtenu de formule : 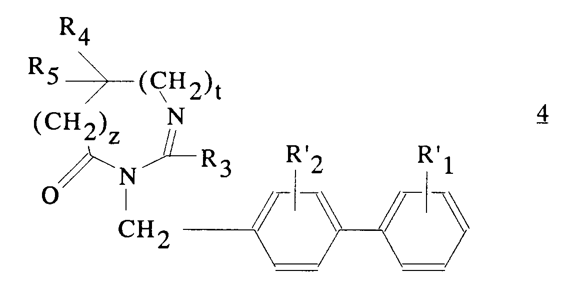 est traité par le réactif de Lawesson [bis(méthoxy-4 phényl)-2,4 dithia-1,3 diphosphénate-2,4 disulfure 2,4] ;
d2) le composé ainsi obtenu en b2) ou en c2) de formule : 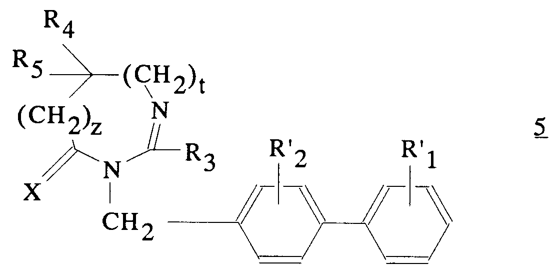 est ensuite traité dans des conditions convenables pour préparer le composé (I) par transformation des groupes R′₁ et/ou R′₂ en, respectivement, les groupes R₁ et/ou R₂ ;
e2) le composé ainsi obtenu est ensuite éventuellement transformé en l'un de ses sels.

3. Procédé selon l'une des revendications 1 ou 2, caractérisé en ce que l'on utilise un composé 3 ou un composé 8 dans lesquels R′₁ est un groupe carboxy estérifié ou un groupe tétrazolyle protégé et R′₂ est l'hydrogène.

4. Procédé selon l'une quelconque des revendications 1 à 3, caractérisé en ce que l'on utilise un composé de formule 2 ou de formule 7 dans lesquels R₄ est le groupe méthyle et R₅ est le groupe cyclohexyle.

5. Procédé selon l'une quelconque des revendications 1 à 4, caractérisé en ce que l'on utilise un composé 2 ou un composé 10 dans lesquels R₃ est un alkyle linéaire en C₁-C₆.

6. Procédé selon l'une quelconque des revendications 1 à 5, caractérisé en ce que l'on utilise un composé 2 ou un composé 9 dans lesquels z = t = 0.

7. Procédé selon la revendication 1, caractérisé en ce que l'on utilise un composé 2 dans lequel R₄ est un groupe méthyle, R₅ est un groupe cyclohexyle, X = O, z = t = 0 et un composé de formule 3 dans laquelle R′₁ est un groupe tétrazolyle protégé et R′₂ est l'hydrogène et en ce que le composé de formule (I) obtenu est n-butyl-2 méthyl-4 cyclohexyl-4 [((tétrazol-5-yl)-2′ biphényl-4-yl) méthyl]-1 imidazoline-2 one-5 ou l'un de ses sels avec des acides ou des bases.

8. Procédé selon la revendication 2, caractérisé en ce que l'on utilise un composé 7 dans lequel R₄ est un groupe méthyle, R₅ est un groupe cyclohexyle, t = z = 0, et un composé de formule 8 dans lequel R′₁ est un groupe tétrazolyle protégé et R′₂ est l'hydrogène et en ce que le composé de formule (I) obtenu est n-butyl-2 méthyl-4 cyclohexyl-4 [((tétrazol-5-yl)-2′ biphényl-4-yl) méthyl]-1 imidazo-line-2 one-5 ou l'un de ses sels avec des acides ou des bases.

9. Procédé pour l'obtention d'un composé de formule : 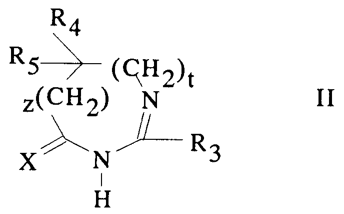 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ représente un alkyle en C₁-C₆, non substitué ou substitué par un ou plusieurs atomes d'halogène ;
- R₅ représente un cycloalkyle ou un cycloalkylméthyle, le cycloalkyle étant en C₃-C₇, non substitué ou substitué par un ou plusieurs atomes d'halogène ;
- ou encore R₄ et R₅ représentent chacun un cyclopropyle ;
- X représente un atome d'oxygène ;
- z et t sont nuls ou l'un est nul et l'autre représente un ;
avec la limitation que R₃ est autre que phényle ou phényle substitué lorsque simultanément z=t=0, R₅ représente un cycloalkyle, caractérisé en ce que l'on fait agir sur un composé de formule : 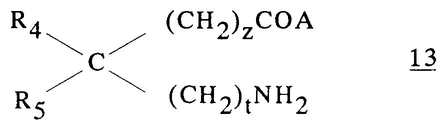 dans laquelle R₄ et R₅ ont les définitions indiquées ci-dessus pour (II), A représente un groupe OH, un groupeNH₂ ou un groupe OR′, R′ étant l'hydrogène ou un alkyle en C₁-C₄, un composé de formule:
R₃ - B 14
dans laquelle R₃ a la définition indiquée ci-dessus pour (II) et B représente :
- un groupement C (OR)₃
- un groupement 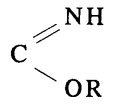 ou
- un groupement CO Hal ;
R étant un alkyle en C₁-C₄ et Hal désignant un atome d'halogène, de préférence le chlore.

10. Procédé selon la revendication 9, pour l'obtention d'un composé de formule : 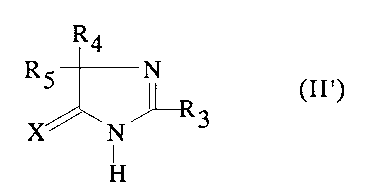 dans laquelle R₃, R₄, R₅ et X ont les définitions données ci-dessus pour (II) dans la revendication 9 et R₃ est un alkyle en C₁-C_{6,}, caractérisé en ce que l'on utilise un composé 13 dans lequel z = t = 0 et un composé 14 dans lequel R₃ est un groupe alkyle en C₁-C₆.

11. Procédé selon la revendication 9, pour l'obtention d'un composé de formule : 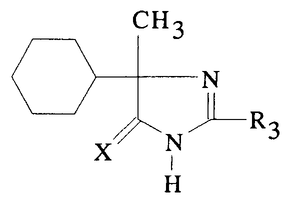 dans laquelle R₃ est un alkyle en C₁-C₆ et X est l'oxygène, caractérisé en ce que l'on utilise un composé 13 dans lequel R₄ est un groupe méthyle, R₅ est un groupe cyclohexyle et z = t = 0, et un composé 14 dans lequel R₃ est un groupe alkyle en C₁-C₆.

12. Procédé selon la revendication 9, pour l'obtention d'un composé de formule : 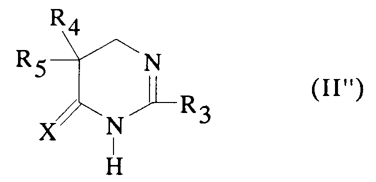 dans laquelle R₃, R₄, R₅ et X ont les définitions données ci-dessus pour (II), dans la revendication 9, caractérisé en ce que l'on utilise un composé 13 dans lequel z = 0 et t = 1.

13. Procédé selon la revendication 9, pour l'obtention d'un composé de formule : 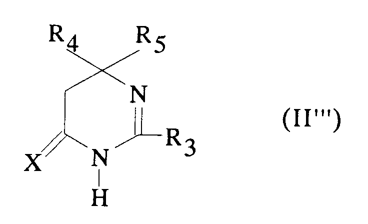 dans laquelle X, R₃, R₄ et R₅ ont les significations indiquées pour (II) dans la revendication 9, caractérisé en ce que l'on utilise un composé 13 dans lequel z = 1 et t = 0.

14. Procédé pour l'obtention d'une composition pharmaceutique caractérisée en ce qu'il consiste à mélanger à titre de principe actif un composé (I) obtenu par le procédé selon l'une quelconque des revendications 1 à 8, avec un véhicule pharmaceutiquement acceptable.

15. Procédé selon la revendication 14, caractérisé en ce qu'il consiste à mélanger le composé (I) avec un autre principe actif choisi parmi les composés béta-bloquants, les diurétiques, les anti-inflammatoires non stéroïdes, les antagonistes calciques, les tranquillisant et avec un véhicule pharmaceutiquement acceptable.
